# EUROPÄISCHE PATENTSCHRIFT

(11) **EP 4 095 529 B1**
(45) Veröffentlichungstag und Bekanntmachung des Hinweises auf die Patenterteilung: **11.12.2024**
(21) Anmeldenummer: 22174497.2
(22) Anmeldetag: 20.05.2022
(51) Int. Cl.: G01N 33/487, G01N 33/497, A61B 5/145

(54) **ANORDNUNG UND VERFAHREN ZUM CHEMISCHEN UNTERSUCHEN EINER PROBE**
APPARATUS AND METHOD FOR CHEMICALLY INSPECTING A SAMPLE
DISPOSITIF ET PROCÉDÉ D'ANALYSE CHIMIQUE D'UN ÉCHANTILLON

(30) Priorität: 28.05.2021 DE 102021113789
(43) Veröffentlichungstag der Anmeldung: 30.11.2022
(60) Teilanmeldung: 24210548.4
(73) Patentinhaber: Dräger Safety AG & Co. KGaA, 23560 Lübeck (DE)
(72) Erfinder: Rattey, Carlo, 23558 Lübeck (DE); Steinmeyer, Stefan, 23558 Lübeck (DE); Kreße, Patrick, 23558 Lübeck (DE)
(74) Vertreter: Meyer-Gramann, Klaus Dieter

(56) Entgegenhaltungen:
- DE-A1- 102020 115 247
- US-A1- 2019 275 518
- US-A1- 2021 128 060

## Beschreibung

Die Erfindung betrifft eine Anordnung und ein Verfahren, welche eine Probe chemisch zu untersuchen vermögen. Die Probe ist insbesondere eine Speichelprobe oder ein Abstrich aus der Nase oder dem Mund und wurde von einem Menschen oder von einem sonstigen Lebewesen genommen. Durch die Untersuchung der Probe soll festgestellt werden, ob die Probe mindestens eine vorgegebene Substanz enthält oder nicht. Die Substanz ist insbesondere eine Droge oder ein Medikament oder auch Mikroben oder Viren.

Ein Drogentestgerät, das sich in einer Hand halten lässt, ist unter der Bezeichnung "Dräger DrugCheck 3000" bekannt geworden. Eine Anordnung mit einem tragbaren Probensammler und einer stationären Basisstation wird unter der Bezeichnung "Dräger DrugTest 5000" angeboten. Bei beiden Geräten gibt ein Proband eine Speichelprobe in einen tragbaren Probensammler ein. Diese Speichelprobe wird auf mehrere verschiedene Drogen und Medikamente untersucht. Ein derartiger Probensammler wird auch in DE 10 2014 001 386 A1 beschrieben. Ein Gerät, welches eine Speichelprobe auf eine "mind-altering substance" zu untersuchen vermag, wird in AU 20 2010 0588 A4 beschrieben.

GB 2483077 A zeigt eine Vorrichtung, mit der sich ein Proband auf Drogen untersuchen lässt. Ein Probensammler (collector 10) vermag eine Probe z.B. aus dem Mund eines Probanden aufzunehmen. Der Probensammler 10 lässt sich in eine Aufnahmeeinheit (disposable cartridge 20) einstecken. Ein Reagens in der Aufnahmeeinheit 20 reagiert chemisch mit der Probe. Ein Sicherungsmechanismus mit einem actuation paddle 33 und einem trigger 36 verhindert, dass die chemische Reaktion ungewollt vorzeitig ausgelöst wird. Durch ein Sichtfenster 38 hindurch lässt sich die Farbe von Teststreifen 28 in der Aufnahmeeinheit 20 ermitteln. Die Aufnahmeeinheit 20 lässt sich in einen Schlitz 51 eines optischen Lesegeräts 50 einsetzen. Das optische Lesegerät 50 vermag die Farbe der Teststreifen 28 durch das Sichtfenster 38 hindurch zu messen und das Ergebnis auf einem Display 53 anzuzeigen.

In DE 10 2014 001 386 A1 wird eine Testvorrichtung 10 mit einem Mischbehälter 1 beschrieben. Im Mischbehälter 1 wird eine zu untersuchende Probe mit einem Reagens vermischt. Hierfür wird die Testvorrichtung 10 geschüttelt. Ein Benutzer kann von außen ein Anzeigeelement 2 visuell auswerten. Das poröse Anzeigeelement 2 nimmt einen Indikator-Farbstoff auf. Durch das Schütteln fließt der Indikator-Farbstoff aus dem Anzeigeelement 2 und löst sich in einem Gemisch aus der Probe und dem Reagens auf. Das Ereignis, dass der Indikator-Farbstoff sich gelöst hat, führt zu einer Entfärbung des Anzeigeelement 2. Dies ist ein Zeichen dafür, dass die Testvorrichtung 10 ausreichend geschüttelt wurde. In einer Ausgestaltung fungiert der Indikator-Farbstoff zugleich als Reagens.

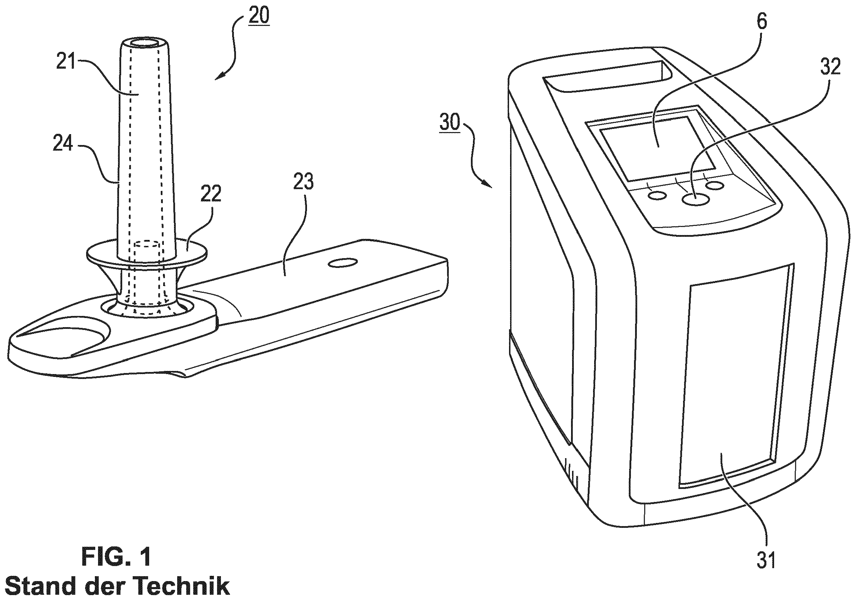

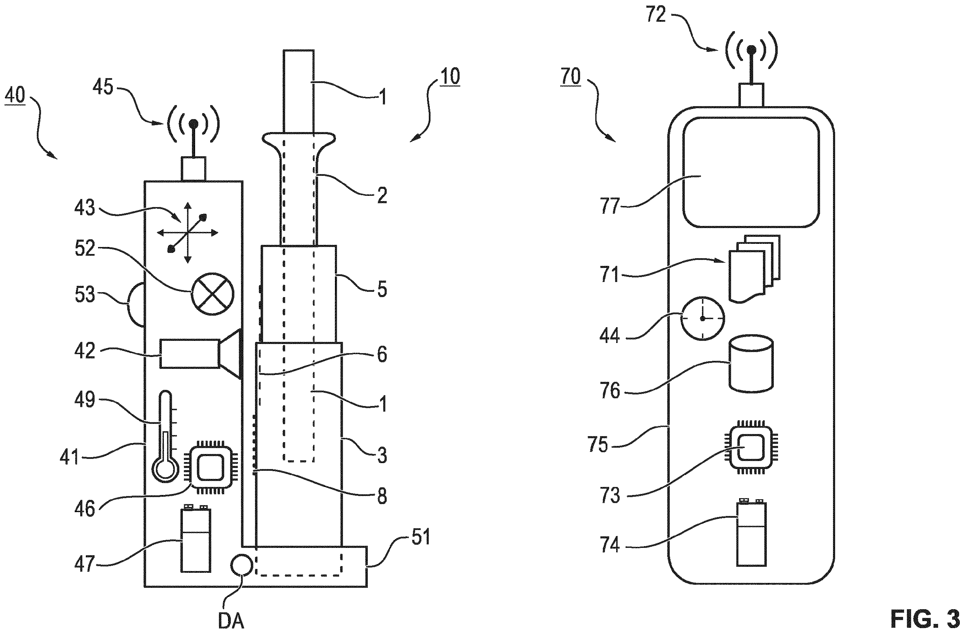

In WO 2007/016691 A2 wird eine Vorrichtung 10 beschrieben, welche eine Probe aufzunehmen und eine Substanz, beispielsweise Bakterien oder Viren oder Bestandteile von diesen in der Probe zu detektieren vermag. Die Vorrichtung 10 lässt sich in eine sample preparation orientation (Fig. 1) und in eine testing orientation (Fig. 3A) bringen. In der sample preparation orientation nimmt ein Probensammler (sample acquisition device 24) der Vorrichtung 10 die Probe auf, und der Probensammler 24 lässt sich in eine Aufnahmeeinheit mit einem Gehäuse 14 einsetzen. Die Probe fließt aus dem Probensammler 24 nach unten in eine Kammer 16 der Aufnahmeeinheit und reagiert dort chemisch mit einem Reagens. Nach einer ausreichend langen Zeitspanne wird die Vorrichtung 10 um 90° gedreht und ist dann in der testing orientation. Die Probe fließt aus der Kammer 16 in ein Testgerät 20 mit einem Sichtfenster 23. Ein Benutzer kann durch das Sichtfenster 23 hindurch eine Farbe im Inneren des Testgeräts 20 wahrnehmen. Die Farbe ist ein Indiz dafür, ob die Substanz in der Probe vorhanden ist oder nicht. US 2021/0128060 zeigt eine Anordnung mit einem Alkoholmessgerät und einem Überwachungssensor.

Der Erfindung liegt die Aufgabe zugrunde, eine Anordnung und ein Verfahren bereitzustellen, welche eine Probe chemisch auf mindestens eine vorgegebene Substanz zu untersuchen vermögen, wobei ermöglicht werden soll, dass die Anordnung und das Verfahren sich mit einer höheren Betriebssicherheit als bekannte Anordnungen benutzen lassen.

Die Aufgabe wird durch eine Anordnung mit den Merkmalen des Anspruchs 1 und durch ein Verfahren mit den Merkmalen des Anspruchs 11 gelöst.

Vorteilhafte Ausgestaltungen sind in den Unteransprüchen angegeben. Vorteilhafte Ausgestaltungen der erfindungsgemäßen Anordnung sind, soweit sinnvoll, auch vorteilhafte Ausgestaltungen des erfindungsgemäßen Verfahrens und umgekehrt.

Die erfindungsgemäße Anordnung und das erfindungsgemäße Verfahren vermögen eine Probe auf mindestens eine Substanz, d.h. auf das Vorhandensein dieser Substanz, zu untersuchen. Die Probe ist insbesondere eine Speichelprobe oder Gaumenprobe oder Nasenprobe. Die Substanz ist insbesondere mindestens eine Droge oder ein Medikament oder mindestens ein Krankheitserreger oder ein Antikörper gegen einen Krankheitserreger oder ein sonstiger Virus.

Die erfindungsgemäße Anordnung umfasst eine Sensoreinheit und eine Überwachungseinheit. Die Sensoreinheit lässt sich mit der Überwachungseinheit mechanisch verbinden, bevorzugt lösbar verbinden. Wenn die Sensoreinheit mit der Überwachungseinheit verbunden ist, so vermag die Sensoreinheit keine Bewegungen relativ zur Überwachungseinheit auszuführen.

Die Sensoreinheit umfasst eine Eingabeeinheit. Diese Eingabeeinheit ermöglicht es, eine zu untersuchende Probe einzugeben. Die Probe ist beispielsweise eine Speichelprobe, die aus dem Mund eines Menschen oder sonstigen Lebewesens entnommen wird, ein Abstrich, der aus der Nase, einem Ohr oder dem Gaumen eines Menschen oder sonstigen Lebewesens entnommen wird, oder eine Probe, die von der Oberfläche eines Gegenstands aufgenommen wird. Beispielsweise soll geprüft werden, ob auf dieser Oberfläche des Gegenstands eine Droge lag oder die Oberfläche mit bestimmten Viren oder Mikroben verunreinigt ist. Die Eingabeeinheit vermag die eingegebene Probe aufzunehmen. Bevorzugt nimmt eine Kammer der Eingabeeinheit die Probe auf.

Die Sensoreinheit umfasst weiterhin einen Substanz-Sensor. Der Substanz-Sensor umfasst mindestens ein chemisches Reagens. Das oder jedes chemische Reagens vermag mit einer Probe, die von der Eingabeeinheit aufgenommen ist, chemisch zu reagieren. Mit anderen Worten: Wenn die Probe mit dem oder einem Reagens in Berührung kommt, so wird automatisch eine chemische Reaktion ausgelöst und läuft dann in der Sensoreinheit ab. Das jeweilige Ergebnis der oder mindestens einer chemischen Reaktion hängt davon ab, ob die Probe die oder mindestens eine vorgegebene Substanz enthält oder nicht - genauer gesagt: ob die Konzentration der Substanz in der Probe oberhalb einer Nachweisgrenze liegt oder nicht. Optional hängt die chemische Reaktion von der Konzentration der Substanz in der Probe ab.

Möglich ist, dass der Substanz-Sensor mehrere Substanzen in der Probe zu detektieren vermag. Bevorzugt ist jedes Reagens jeweils mindestens einer Substanz, auf welche die Probe zu untersuchen ist, zugeordnet. Bevorzugt umfasst der Substanz-Sensor daher mehr als ein Reagens. Möglich ist, dass dasselbe Reagens verschiedenen vorgegebenen Substanzen, auf die eine eingegebene Probe zu untersuchen ist, zugeordnet ist. Möglich ist auch, dass derselben Substanz ein erstes und ein zweites Reagens zugeordnet sind, die sich voneinander unterscheiden.

Die oder eine Substanz ist insbesondere eine Droge, beispielsweise Cannabis, ein Amphetamin, ein Opiat oder Kokain, oder ein Medikament oder auch ein Krankheitserreger oder ein Antikörper, der das Vorhandensein eines Krankheitserregers im Körper des Probanden anzeigt. Ein als Substanz zu detektierendes Medikament ist insbesondere aus der Klasse der Benzodiazepine oder der Klasse der synthetischen Opioide, ist z.B. ein Oxycodon.

Der Substanz-Sensor umfasst weiterhin mindestens einen Indikator. Der oder jeder Indikator ist jeweils einer zu detektierenden Substanz zugeordnet und vermag das jeweilige Ergebnis einer chemischen Reaktion des Reagens, das dieser Substanz zugeordnet ist, mit der Probe visuell anzuzeigen. Beispielsweise verfärbt sich der Indikator abhängig davon, ob die oder eine Substanz, welcher das Reagens zugeordnet ist, in der Probe enthalten ist oder nicht. Beispielsweise verfärbt sich der Indikator nur dann, wenn die Substanz vorhanden ist, oder nur dann, wenn die Substanz nicht vorhanden ist, oder verfärbt sich unterschiedlich, je nachdem ob die Substanz vorhanden ist oder nicht.

Die Überwachungseinheit umfasst mindestens einen Überwachungs-Sensor, optional mehrere Überwachungs-Sensoren. In der Erfindung vermag der oder mindestens ein Überwachungs-Sensor jeweils ein Maß für eine Bewegung der Sensoreinheit im Raum zu messen, und zwar mindestens dann, wenn diese Sensoreinheit mit der Überwachungseinheit verbunden ist.

In vielen Fällen lässt die Sensoreinheit sich dank der Überwachungseinheit ohne ein Bauteil realisieren, welches elektrische Energie benötigt. Insbesondere ist es nicht erforderlich, die Sensoreinheit selber mit einem Überwachungs-Sensor oder einer Kommunikationseinheit zu versehen. Ein Überwachungs-Sensor und eine Kommunikationseinheit benötigen in der Regel elektrische Energie. Weil die Sensoreinheit sich mechanisch mit der Überwachungseinheit verbinden lässt und diese Überwachungseinheit mindestens einen Überwachungs-Sensor umfasst, wird trotzdem die Sensoreinheit selber überwacht, während sie mit der Überwachungseinheit verbunden ist, nämlich von mindestens einem Überwachungs-Sensor der Überwachungseinheit, optional von mehreren Überwachungs-Sensoren. Möglich ist, dass die Sensoreinheit keinen elektrischen Verbraucher umfasst. Dann benötigt die Sensoreinheit weder eine eigene Spannungsversorgungseinheit noch eine Verbindung zu einem stationären Spannungsversorgungsnetz. Diese Ausgestaltung ermöglicht es in vielen Fällen, eine relativ kleine und / oder leichte Sensoreinheit bereitzustellen.

Die Sensoreinheit braucht nicht als elektrisches Gerät zugelassen zu werden. Dies erleichtert in vielen Fällen die Konstruktion der Sensoreinheit.

Die Überwachungseinheit verbraucht in der Regel relativ wenig elektrische Energie, nämlich insbesondere die zum Betrieb der Überwachungs-Sensoren benötigte elektrische Energie. Weil die Überwachungseinheit nur wenig elektrische Energie verbraucht, kann eine eigene Spannungsversorgungseinheit der Überwachungseinheit relativ klein ausfallen oder für relativ lange Zeit elektrische Energie liefern. Dieses Merkmal erleichtert es, die erfindungsgemäße Anordnung mobil zu verwenden, beispielsweise um an einer Straße oder auf einem Parkplatz einen Fahrer eines Kraftfahrzeugs zu überprüfen. Nicht erforderlich ist es, die erfindungsgemäße Anordnung mit einem stationären Spannungsversorgungsnetz zu verbinden, um die Probe aufzunehmen und auszuwerten. Eine Verbindung ist insbesondere nicht erforderlich, während die Sensoreinheit chemisch mit einer aufgenommenen Probe reagiert.

Gemäß der Erfindung vermag der oder mindestens ein Überwachungs-Sensor ein Maß für eine Bewegung der Sensoreinheit im Raum zu messen, wenn die Sensoreinheit mit der Überwachungseinheit verbunden ist. Erfindungsgemäß vermag die Sensoreinheit dann, wenn sie mit der Überwachungseinheit verbunden ist, keine Bewegung relativ zu Überwachungseinheit auszuführen. Daher reicht es aus, wenn der Überwachungs-Sensor seine eigene Bewegung im Raum misst. Diese eigene Bewegung des Überwachungs-Sensors stimmt dank der mechanischen Verbindung mit der Bewegung der Sensoreinheit im Raum überein. Möglich, aber dank der mechanischen Verbindung nicht erforderlich ist, direkt die Bewegung der Sensoreinheit im Raum zu messen. Ein Signal des Überwachungs-Sensors korreliert also mit der Bewegung der Sensoreinheit im Raum.

Der Überwachungs-Sensor misst beispielsweise, wie lange die Sensoreinheit im Raum bewegt worden ist, wie lange sie mit einer Minimal-Geschwindigkeit oder einer Minimal-Beschleunigung bewegt worden ist und / oder die maximale Beschleunigung der Sensoreinheit im Raum. Oder der Überwachungs-Sensor misst, welchen Weg die Sensoreinheit bei der Bewegung im Raum insgesamt oder in einem vorgegebenen Zeitraum zurückgelegt hat.

In vielen Fällen ist es erforderlich, dass die Sensoreinheit mitsamt der aufgenommenen Probe im Raum bewegt wird, damit die gewünschte chemische Reaktion der Probe mit dem oder mindestens einem Reagens stattfindet und damit das Ergebnis der Reaktion zuverlässig anzeigt, ob und / oder mit welcher Konzentration die Substanz in der Probe vorhanden ist. Insbesondere ist es in vielen Fällen erforderlich, die Sensoreinheit zu schütteln. Oft findet bei einer fehlenden oder unzureichenden Bewegung die chemische Reaktion nicht statt, und dann kann das Vorhandensein der Substanz nicht sicher genug ausgeschlossen werden. Andererseits ist es in vielen Fällen erforderlich, dass die Sensoreinheit für eine bestimmte Zeitspanne nicht bewegt wird, damit die chemische Reaktion in Ruhe ablaufen kann. In vielen Fällen sind beide Anforderungen einzuhalten, also sowohl eine ausreichende Bewegung als auch eine ausreichende Ruhepause der Sensoreinheit.

Dank des Überwachungs-Sensors lässt sich die gemessene Bewegung der Sensoreinheit automatisch mit mindestens einem vorgegebenen Kriterium vergleichen. Das oder jedes Kriterium spezifiziert beispielsweise einen Bereich für die Dauer der Bewegung oder die Beschleunigung oder die Amplitude, die bei der Bewegung der Sensoreinheit erzielt werden soll, oder auch die Dauer einer Ruhephase, in der die Sensoreinheit nicht bewegt werden soll, oder beides. Eine Auswerteeinheit vermag dann automatisch zu entscheiden, ob die Sensoreinheit korrekt im Raum bewegt worden ist oder nicht. Dadurch lässt sich insbesondere automatisch entscheiden, ob das Ergebnis, das die Sensoreinheit bei der Untersuchung der Probe liefert, gültig ist oder nicht.

Besonders vorteilhaft ist die Erfindung dann, wenn die Anordnung von einem Menschen geschüttelt wird und nicht von einem Rüttler oder einer sonstigen Maschine. Die Erfindung erspart es diese Menschen, selber zu überwachen, auf welche Weise er sie Anordnung bewegt oder hält.

Erfindungsgemäß beeinflusst die chemische Reaktion zwischen der Probe und dem Reagens den oder mindestens einen Indikator, und der Indikator zeigt das Ergebnis der Reaktion visuell an. In vielen Fällen ist es nicht erforderlich, die Sensoreinheit während der gesamten Dauer der chemischen Reaktion im Raum zu bewegen. Vielmehr reicht es in vielen Fällen aus, die Sensoreinheit in einer Anfangs-Zeitspanne im Raum zu bewegen, insbesondere um die Probe mit dem oder jedem Reagens zu vermischen, und sie anschließend unbewegt zu lassen. Dank der Erfindung braucht nicht ein Benutzer den Indikator visuell zu überwachen, um zu wissen, wann die Bewegung der Sensoreinheit beendet werden kann.

Erfindungsgemäß lässt sich die Sensoreinheit mechanisch mit der Überwachungseinheit verbinden. Bevorzugt wird nach Herstellung dieser Verbindung eine tragbare Mess-Anordnung bestehend aus der Sensoreinheit und der Überwachungseinheit gebildet. Ein Mensch kann diese Mess-Anordnung schütteln. Um die tragbare Mess-Anordnung zu schütteln, braucht der Mensch nur relativ wenig mechanische Arbeit aufzubringen, was diesen Menschen daher nur relativ wenig ermüdet. In vielen Fällen löst dieses Schütteln mindestens eine chemische Reaktion zwischen einem Reagens der Sensoreinheit und der Probe, die von der Eingabeeinheit aufgenommen ist, aus, optional mehrere chemische Reaktionen. In vielen Fällen wird die oder jede chemische Reaktion nur dann ausgelöst, wenn die Mess-Anordnung und damit die Sensoreinheit mit der Probe ausreichend lange geschüttelt wird. Weil ein Mensch diese Mess-Anordnung schütteln kann, wird in vielen Fällen die Notwendigkeit vermieden, dass ein Gerät umfassend einen Rüttler oder Schüttler die Sensoreinheit schüttelt. Ein solches Gerät muss bereitgestellt werden und benötigt in der Regel wesentlich mehr elektrische Energie als die Überwachungseinheit und / oder ist schwerer, weswegen das Gerät sich bei vielen Anwendungen nur kurz oder überhaupt nicht einsetzen lässt. Insbesondere lässt sich eine Mess-Anordnung, welche ein Mensch schütteln kann, häufig mobil einsetzen, beispielsweise um einen Fahrer eines Kraftfahrzeugs an einer Straße oder auf einem Parkplatz auf Drogen zu untersuchen.

Besonders bevorzugt kann ein Mensch die Mess-Anordnung in einer Hand halten und schütteln.

Bevorzugt lässt sich die Überwachungseinheit mit der Sensoreinheit lösbar verbinden, besonders bevorzugt dieselbe Überwachungseinheit nacheinander lösbar mit verschiedenen Sensoreinheiten, wobei jede verwendete Sensoreinheit jeweils eine Eingabeeinheit und einen Substanz-Sensor umfasst. Diese Ausgestaltung ermöglicht es, eine Sensoreinheit wieder von der Überwachungseinheit zu lösen und dieselbe Überwachungseinheit mehrmals zu verwenden, nämlich nacheinander mit verschiedenen Sensoreinheiten. Dies spart Material und insbesondere Abfall ein.

Erfindungsgemäß lässt sich die Sensoreinheit mit der Überwachungseinheit verbinden. In einer Ausgestaltung wird als die Überwachungseinheit ein tragbarer Rechner verwendet, insbesondere ein Smartphone oder ein Tablet. Ein solcher tragbarer Rechner besitzt häufig bereits diejenigen Sensoren, welche erfindungsgemäß oder gemäß verschiedenen Ausgestaltungen der Erfindung als Überwachungs-Sensoren verwendet werden oder sich bevorzugt verwenden lassen, sowie eine eigene Spannungsversorgungseinheit, insbesondere einen Akkumulator. Insbesondere umfasst ein solcher tragbarer Rechner häufig eine Systemuhr sowie einen Beschleunigungs-Sensor oder sonstigen Bewegungs-Sensor, der ein Maß für die Bewegung des Rechners im Raum zu messen vermag. Dieser tragbare Rechner vermag damit auch ein Maße für die Bewegung der Sensoreinheit im Raum zu messen. Außerdem umfasst ein solcher tragbarer Rechner häufig eine Kamera, wobei diese Kamera dann den Zustand des Indikators zu erfassen vermag, wenn die Sensoreinheit geeignet mit der Überwachungseinheit in Form des tragbaren Rechners verbunden ist.

Die Mess-Anordnung umfasst gemäß dieser Ausgestaltung weiterhin einen mechanischen Adapter. Mithilfe dieses Adapters lässt die Sensoreinheit sich lösbar mit dem tragbaren Rechner verbunden oder ist lösbar mit dem tragbaren Rechner verbunden. Dadurch wird eine tragbare Mess-Anordnung bereitgestellt, die ein Mensch halten und schütteln kann. Der Adapter kann als ein rein passives mechanisches Bauteil ausgestaltet sein. Möglich ist auch, als Überwachungseinheit ein anderes Gerät als einen tragbaren Rechner zu verwenden und dieses Gerät mittels des Adapters mit der Sensoreinheit zu verbinden, bevorzugt lösbar zu verbinden.

Die Ausgestaltung mit dem Adapter erspart in vielen Fällen die Notwendigkeit, eine spezielle Überwachungseinheit bereitstellen zu müssen. Ausreichend ist es, einen Adapter bereitzustellen, der auf der einen Seite an die Abmessungen eines handelsüblichen Smartphones oder sonstigen tragbaren Rechners oder sonstigen Geräts und auf der anderen Seite an die Abmessungen der Sensoreinheit angepasst ist.

Der Adapter umfasst bevorzugt eine überwachungsseitige Koppeleinheit, die sich mit der Überwachungseinheit in Form des tragbaren Rechners lösbar koppeln lässt, sowie eine sensorseitige Koppeleinheit, die sich mit der Sensoreinheit lösbar koppeln lässt. Derselbe Adapter lässt sich nacheinander für verschiedene Sensoreinheiten und bevorzugt auch für verschiedene Überwachungseinheiten verwenden.

In einer Ausgestaltung lässt sich eine Abmessung der überwachungsseitigen Koppeleinheit des Adapters verändern. Dadurch wird es ermöglicht, den Adapter an die Abmessungen eines tragbaren Rechners, der als die Überwachungseinheit verwendet wird, anzupassen. Dieselbe Sensoreinheit lässt sich wahlweise mit einem von mehreren tragbaren Rechnern als der Überwachungseinheit verbinden. Derselbe Adapter lässt sich wahlweise mit einem von mehreren möglichen tragbaren Rechnern als der Überwachungseinheit verbinden. Nicht erforderlich ist es, verschiedene Adapter für unterschiedliche Sensoreinheiten oder Überwachungseinheiten vorzusehen.

In einer Realisierungsform ist der Adapter so ausgestaltet, dass sich der Abstand zwischen den beiden Koppelstellen verändern lässt. Dadurch lässt sich auch der Abstand zwischen der Überwachungseinheit und der Sensoreinheit verändern. Allgemein lässt sich die eine Koppelstelle relativ zu der anderen Koppelstelle verschieben oder anderweitig bewegen. Das Merkmal, dass die eine Koppelstelle relativ zu der anderen Koppelstelle bewegbar ist, erleichtert es, die Sensoreinheit dergestalt relativ zu Überwachungseinheit zu positionieren, dass folgendes bewirkt wird: Ein Überwachungs-Sensor der Überwachungseinheit in Form einer Kamera des tragbaren Rechners ist auf den oder mindestens einen Indikator der Sensoreinheit gerichtet, und dieser Indikator liegt in dem Bereich, den die Kamera scharf abzubilden vermag. Ein Bild, welches die Kamera erzeugt hat, zeigt daher den Indikator. Außerdem lässt sich dank dieses Adapters Folgendes mit größerer Sicherheit bewirken: Die verbundene Sensoreinheit vermag keine Bewegung relativ zur Überwachungseinheit auszuführen. Oft ist es dank des Adapters nicht erforderlich, die Sensoreinheit hinsichtlich ihrer Geometrie an die Überwachungseinheit oder umgekehrt anzupassen.

Erfindungsgemäß zeigt der oder jeder Indikator des Substanz-Sensors visuell an, welches Ergebnis die chemische Reaktion zwischen der Probe und dem zugeordneten Reagens erbracht hat, wobei das Ergebnis und damit die visuelle Anzeige davon abhängen, ob die Probe die Substanz, welcher das Reagens zugeordnet ist, enthält oder nicht. Beispielsweise verfärbt sich der Indikator abhängig davon, ob die Probe die Substanz enthält oder nicht, oder nimmt eine Farbe an, die von dem Vorhandensein oder Nichtvorhandensein der Substanz abhängt. Ein Bild, welches die Kamera erzeugt hat und welches diesen Indikator zeigt, zeigt auch das visuell wahrnehmbare Ergebnis der chemischen Reaktion. Indem dieses Bild ausgewertet wird, lässt sich feststellen, ob die Substanz in der Probe enthalten ist oder nicht.

Möglich ist, dass ein Bildauswerte-Programm das oder mindestens ein Bild der Kamera automatisch auswertet. Bevorzugt identifiziert das Bildauswerte-Programm das Abbild des oder eines Indikators im Bild und ermittelt den Zustand des identifizierten Indikators. Dieses Bildauswerte-Programm kann auf einem räumlich entfernten Rechner oder auf der Überwachungseinheit selber installiert sein. Falls als Überwachungseinheit ein tragbarer Rechner verwendet wird, so umfasst dieser tragbare Rechner in vielen Fällen bereits die erforderlichen Prozessoren und Datenspeicher, um ein Bildauswerte-Programm zu installieren. Möglich ist auch, dass ein Mensch das Bild auswertet und das Ergebnis der Auswertung eingibt oder das Ergebnis einer automatischen Auswertung überprüft. In allen Fällen ist es möglich, dass die Bildauswertung räumlich entfernt von der Sensoreinheit und der Überwachungseinheit durchgeführt wird, optional auch mit einem zeitlichen Versatz. Dies ermöglicht eine kleinere Überwachungseinheit.

Erfindungsgemäß umfasst der Substanz-Sensor mindestens einen Indikator, der ein Ergebnis einer chemischen Reaktion zwischen dem Reagens, dem der Indikator zugeordnet ist, und der Probe visuell anzeigt. In einer Ausgestaltung umfasst die Sensoreinheit eine Anzeigefläche, beispielsweise eine durchsichtige Scheibe. Wenigstens ein Teil des oder jedes Indikators ist auf oder hinter dieser Anzeigefläche zu sehen. Dadurch ist es möglich, auf einen Blick jeden Indikator visuell wahrzunehmen. Die optionale Kamera der Überwachungseinheit kann mindestens ein Bild von dieser Anzeigefläche erzeugen, wenn die Sensoreinheit mit der Überwachungseinheit verbunden ist, und dieses Bild zeigt die Anzeigefläche und damit alle Indikatoren.

In einer Realisierungsform ist die Kamera der Überwachungseinheit so ausgestaltet, dass die Kamera eine Abfolge von Bildern erzeugt, wobei diese Abfolge zeigt, wie sich der oder mindestens ein, bevorzugt jeder Indikator im Laufe der Zeit visuell verändert. In manchen Anwendungen korreliert der zeitliche Verlauf des Aussehens eines Indikators mit der Konzentration der zugeordneten Substanz in der Probe. Indem die Bilder-Abfolge ausgewertet wird, lässt sich in vielen Fällen daher wenigstens näherungsweise die Konzentration dieser Substanz in der Probe ermitteln.

Die Ausgestaltung mit der Kamera ermöglicht es, das Ergebnis einer chemischen Reaktion zu erfassen, auch aus der Ferne zu erfassen, ohne dass die Sensoreinheit ein Untersuchungsergebnis zu übermitteln braucht. Dadurch lässt sich die Sensoreinheit ohne eine Spannungsversorgungseinheit und ohne eine Kommunikationseinheit realisieren. Weiterhin ist keine kabelgebundene Datenverbindung zwischen der Sensoreinheit und der Überwachungseinheit erforderlich. Die Sensoreinheit kann als ein passives mechanisches Bauteil aufgebaut sein, welches das oder jedes erforderliche Reagens enthält, aber keinen elektrischen Verbraucher. Dank der Kamera der Überwachungseinheit ist es auch nicht erforderlich, dass ein Mensch den oder jeden Indikator inspiziert und die angezeigten Ergebnisse notiert und / oder manuell in die Überwachungseinheit eingibt. Vielmehr reicht es aus, Bilder der Kamera an einen räumlich entfernten Empfänger zu übermitteln.

In einer Fortbildung der Ausgestaltung mit der Kamera umfasst die Anordnung ein Gehäuse. Der oben erwähnte Adapter mit den beiden Koppelstellen kann als dieses Gehäuse verwendet werden. Wenn die Sensoreinheit mit der Überwachungseinheit verbunden ist, umschließt dieses Gehäuse einen Raum. Bevorzugt umschließt das Gehäuse den Raum lichtdicht. Der umschlossene Raum befindet sich zwischen der Sensoreinheit und der Überwachungseinheit. Die Kamera der Überwachungseinheit und der oder jeder Indikator der Sensoreinheit grenzen an diesen Raum an.

Die Ausgestaltung mit dem Gehäuse, welches einen Raum umschließt, erhöht die Sicherheit der Ausgestaltung mit der Kamera. Das Gehäuse reduziert das Risiko, dass natürliches oder künstliches Umgebungslicht auf einen Indikator fällt und eine Auswertung des Bildes, welches die Kamera erzeugt hat und diesen Indikator zeigt, zu einem falschen Ergebnis führt oder dass ein Indikator aufgrund eines Lichteinfalls nicht im Bild sichtbar ist. Das Gehäuse erleichtert es, eine automatische Bildauswertung zu justieren. Das Gehäuse führt nämlich zu gleichbleibenden Lichtverhältnissen im umschlossenen Raum unabhängig von der Beleuchtung in der Umgebung.

In einer weiteren Fortbildung der Ausgestaltung mit der Kamera umfasst die Überwachungseinheit eine Lichtquelle. Diese Lichtquelle vermag Licht im sichtbaren Bereich oder auch im Infrarotbereich oder im ultravioletten Bereich auszustrahlen. Wenn die Sensoreinheit mit der Überwachungseinheit verbunden ist, fällt wenigstens ein Teil des Lichts, welches die Lichtquelle ausgestrahlt hat, auf den oder mindestens einen Indikator. Bevorzugt umfasst die Sensoreinheit eine Anzeigefläche, wobei der oder jeder Indikator auf oder hinter der Anzeigefläche wenigstens teilweise sichtbar ist. Das ausgestrahlte Licht fällt auf diese Anzeigefläche. Dank der Ausgestaltung mit der Lichtquelle vermag die Kamera auch dann ein Bild zu erzeugen, welches den oder mindestens einen Indikator zeigt, wenn wenig Umgebungslicht vorhanden ist, beispielsweise bei Nacht oder in einem dunklen Raum.

Die Ausgestaltung mit dem Gehäuse und die Ausgestaltung mit der Lichtquelle lassen sich kombinieren. Die Lichtquelle leuchtet in den Raum, der vom Gehäuse lichtdicht umschlossen ist. Bei dieser Kombination herrschen in dem umschlossenen Raum stets die gleichen oder wenigstens einstellbare Lichtverhältnisse. Die Ausgestaltung mit dem Gehäuse lässt sich auch mit der Ausgestaltung mit dem Adapter kombinieren.

In einer Ausgestaltung ist die Kamera dazu ausgestaltet, nach Erhalt eines Auslösesignals eine Abfolge von Bildern zu erzeugen. Bevorzugt zeigt diese Abfolge, wie sich der oder mindestens ein Indikator im Laufe der Zeit visuell verändert. Bevorzugt erzeugt die Kamera die Bilder mit einer vorgegebenen Bildaufnahmefrequenz. Bevorzugt erzeugt ein signalverarbeitendes Steuergerät der Überwachungseinheit das Auslösesignal. Die Kamera beendet den Vorgang, die Bilder zu erzeugen, wenn eine vorgegebene Zeitspanne verstrichen ist oder wenn die Kamera ein Beendigungssignal von dem Steuergerät der Überwachungseinheit erhalten hat. Dadurch werden in vielen Fällen nicht mehr Bilder als nötig erzeugt, was Speicherplatz und elektrische Energie einspart.

Gemäß der Erfindung vermag der oder mindestens ein Überwachungs-Sensor ein Maß für die Bewegung der Sensoreinheit im Raum zu messen, beispielsweise die Beschleunigung. In einer Ausgestaltung vergleicht eine signalverarbeitende Auswerteeinheit automatisch ein Signal dieses Überwachungs-Sensors mit mindestens einem vorgegebenen Kriterium, wobei das oder jedes Kriterium in einer rechnerauswertbaren Form abgespeichert ist. Das Kriterium spezifiziert eine Anforderung an die Bewegung, beispielsweise die kleinste oder größte maximale Dauer oder die minimale oder maximale Beschleunigung. In einer Ausgestaltung erzeugt die Auswerteeinheit abhängig vom Vergleich zwischen dem Signal des Überwachungs-Sensors und dem Kriterium eine Anweisung an einen Benutzer, wobei diese Anweisung eine zukünftige Behandlung der Sensoreinheit spezifiziert. Beispielsweise legt diese Anweisung fest, dass die Sensoreinheit weiter zu bewegen ist oder dass nunmehr die Bewegung der Sensoreinheit zu beenden ist. Bevorzugt wird diese Anweisung in einer von einem Menschen wahrnehmbaren Form ausgegeben. Diese Ausgestaltung unterstützt einen Benutzer dabei, die Sensoreinheit korrekt zu bewegen. In vielen Fällen lässt sich der Fehler, dass die Sensoreinheit bislang zu kurz bewegt worden ist, durch eine entsprechende Anweisung an den Benutzer noch korrigieren. Ohne eine solche Anweisung würde hingegen die Untersuchung der Probe möglicherweise ein ungültiges Ergebnis liefern.

Möglich ist auch, dass ein spezielles Gerät, beispielsweise ein Rückschlag oder ein Schüttler, die Anordnung mit der Sensoreinheit und der Überwachungseinheit im Raum zu bewegen vermag. Ein Steuergerät steuert dieses spezielle Gerät an. Das Steuergerät empfängt ein Signal von der Überwachungseinheit und vergleicht dieses Signal mit dem oder mindestens einem vorgegebenen Kriterium, welches eine Anforderung an die Bewegung der Sensoreinheit spezifiziert. Abhängig vom Ergebnis dieses Vergleichs steuert das Steuergerät das spezielle Gerät an und bewirkt insbesondere, dass das Gerät die Bewegung der Anordnung fortsetzt oder beendet.

In einer Ausgestaltung umfasst der oder ein Überwachungs-Sensor einen Beschleunigungs-Sensor oder ist als Beschleunigungs-Sensor ausgestaltet. Oft umfasst ein tragbarer Rechner, der als Überwachungseinheit verwendet wird, bereits einen solchen Beschleunigungs-Sensor. Der Beschleunigungs-Sensor vermag den zeitlichen Verlauf einer Beschleunigung im Raum zu messen, welche auf die Überwachungseinheit einwirkt, beispielsweise weil die Überwachungseinheit geschüttelt oder ruhig gehalten wird. Zu jedem Abtast-Zeitpunkt liefert der Beschleunigungs-Sensor also jeweils drei Messwerte, welche zusammen einen Vektor im dreidimensionalen Raum definieren. Selbstverständlich ist es möglich, dass der Überwachungs-Sensor zu mindestens einem Abtast-Zeitpunkt feststellt, dass keine Beschleunigung auf die Überwachungseinheit einwirkt, der Überwachungs-Sensor also das Ausbleiben einer Beschleunigung misst. Die Beschleunigung fungiert als ein Maß für die Bewegung im Raum. Die Abfolge mit jeweils drei Messwerten fungiert als ein Signal des Überwachungs-Sensors.

Gemäß der Erfindung vermag mindestens ein Überwachungs-Sensor ein Maß für eine Bewegung der Sensoreinheit im Raum zu messen. Dieser Überwachungs-Sensor wird nachfolgend als Bewegungs-Sensor bezeichnet. Ein Beschleunigungs-Sensor ist eine Ausgestaltung eines Bewegungs-Sensors.

Erfindungsgemäß lässt sich die Sensoreinheit mit der Überwachungseinheit verbinden, bevorzugt lösbar verbinden. Wenn diese Verbindung hergestellt ist, vermag die Sensoreinheit idealerweise überhaupt keine Bewegung relativ zur Überwachungseinheit auszuführen. Mindestens vermag die Sensoreinheit, die mit der Überwachungseinheit verbunden ist, keine solchen Bewegungen relativ zur Überwachungseinheit auszuführen, dass ein Bewegungs-Signal des Bewegungs-Sensors zwar für die Überwachungseinheit, aber nicht für die Sensoreinheit gültig sind. Wegen der mechanischen Verbindung misst der Bewegungs-Sensor den zeitlichen Verlauf einer Bewegung der Sensoreinheit, z.B. einer Beschleunigung, die auf die Sensoreinheit einwirkt.

Die Ausgestaltung mit dem Bewegungs-Sensor ist insbesondere dann von Vorteil, wenn die Sensoreinheit mit einer aufgenommenen Probe geschüttelt werden muss, damit die oder jede chemische Reaktion eines Reagens des Sensors mit der Probe ausgelöst wird und korrekt abläuft. Durch dieses Schütteln wird die Probe mit dem Reagens vermischt. Nur dann, wenn die Sensoreinheit ausreichend stark und / oder ausreichend lange geschüttelt worden ist, vermag sie ein gültiges Ergebnis zu liefern. Dank des Bewegungs-Sensors ist es nicht erforderlich, dass ein Mensch den Vorgang überwacht und protokolliert, die Sensoreinheit zu schütteln. Weil der Bewegungs-Sensor gemäß dieser Ausgestaltung ein Bestandteil der Überwachungseinheit ist und die Sensoreinheit sich mit der Überwachungseinheit verbinden lässt, ist es nicht erforderlich, die Sensoreinheit selber mit einem Bewegungs-Sensor zu versehen. Daher braucht die Sensoreinheit auch keine Spannungsversorgungseinheit aufzuweisen. Dieses Merkmal ist insbesondere dann von Vorteil, wenn dieselbe Überwachungseinheit nacheinander mit mehreren Sensoreinheiten verbunden wird, um nacheinander mehrere Proben zu untersuchen.

Der tatsächliche zeitliche Verlauf der Bewegung im Raum, den die Sensoreinheit ausführt, lässt sich abspeichern und / oder mit einem vorgegebenen zeitlichen Sollverlauf vergleichen. Dadurch lässt sich überprüfen, ob die Sensoreinheit korrekt geschüttelt worden ist oder nicht. Diese Überprüfung lässt sich auch im nachherein und / oder aus der Entfernung durchführen. Eine solche Überprüfung ist insbesondere dann in vielen Fällen von Vorteil oder sogar erforderlich, wenn einem Probanden ein Konsum einer illegalen Droge oder der Missbrauch eines Medikaments nachgewiesen werden soll oder wenn sichergestellt werden soll, dass ein Proband tatsächlich keine unerlaubte Droge zu sich genommen hat und / oder nicht ein Medikament missbräuchlich eingenommen hat, und sichergestellt werden muss, dass ein entsprechendes positives oder negatives Untersuchungsergebnis tatsächlich gültig und richtig ist. Das Entsprechende gilt für die Prüfung, ob ein Proband mit einem Virus infiziert ist.

In einer Ausgestaltung werden der gemessene tatsächliche zeitliche Verlauf der Bewegung, z.B. der Beschleunigung, sowie das Ergebnis, welche die Sensoreinheit bei der Untersuchung der Probe erzielt hat, manipulationssicher abgespeichert, also so, dass eine nachträgliche Veränderung nicht mehr oder wenigstens nicht mehr unerkannt möglich ist. Dadurch ist das Ergebnis der Sensoreinheit gerichtsfest verwertbar.

In einer Weiterbildung der Ausgestaltung mit dem Bewegungs-Sensor wird das Signal vom Bewegungs-Sensor verwendet, um den oben bereits beschriebenen Vorgang auszulösen, dass die Kamera eine Abfolge von Bildern erzeugt. Der Vorgang, dass die Kamera die Abfolge erzeugt, wird abhängig vom Signal des Bewegungs-Sensors ausgelöst. In einer Realisierungsform wird der Vorgang, dass die Kamera die Abfolge erzeugt, ausgelöst, nachdem die Mess-Anordnung umfassend die Sensoreinheit und die Überwachungseinheit bewegt worden ist und anschließend die Mess-Anordnung eine vorgegebene Zeitspanne lang nicht bewegt worden ist. In vielen Fällen setzt nämlich nach dieser Zeitspanne eine chemische Reaktion ein, die zu einer allmählichen und visuell wahrnehmbaren Veränderung des oder mindestens eines Indikators führt. Diese chemische Reaktion kann von der Konzentration einer Substanz in der Probe abhängen. Die Abfolge von Bildern zeigt den Verlauf dieser chemischen Reaktion in vielen Fällen besser an als ein einzelnes Bild. Andererseits führt die Ausgestaltung, dass das Signal des Bewegungs-Sensors dafür verwendet werden, das Auslösesignal und / oder das Beendigungssignal zu erzeugen, in vielen Fällen dazu, dass die Kamera nur dann Bilder erzeugt, wenn tatsächlich eine chemische Reaktion stattfindet und sichtbar ist. Dies spart elektrische Energie zum Betrieb der Kamera und Speicherplatz für die erzeugten Bilder ein.

In einer Weiterbildung der Ausgestaltung mit dem Bewegungs-Sensor umfasst die Anordnung ein Auswerte-Programm, also Software, beispielsweise eine App für einen tragbaren Rechner, der als die Überwachungseinheit verwendet wird. Dieses Auswerte-Programm vermag das Signal des Bewegungs-Sensors auszuwerten. Aufgrund dieser Auswertung vermag das Auswerte-Programm automatisch zu entscheiden, ob der tatsächliche zeitliche Verlauf einer Bewegung im Raum, die auf die Sensoreinheit einwirkt, ein vorgegebenes Kriterium erfüllt oder nicht. Bevorzugt wird dieses Kriterium wie folgt vorgegeben: Wenn der tatsächliche zeitliche Verlauf der Bewegung, welche die Sensoreinheit im Raum ausführt, das Kriterium erfüllt, so ist die Probe ausreichend mit dem oder jedem Reagens vermischt, und die Sensoreinheit liefert gültige Ergebnisse. Bevorzugt ist das Kriterium, ob der tatsächliche zeitliche Verlauf bis auf eine vorgegebene Toleranz gleich einem vorgegebenen zeitlichen Sollverlauf ist oder nicht. Das Kriterium kann weiterhin die Dauer einer Ruhephase spezifizieren, wobei diese Ruhephase nach einer Bewegung, insbesondere nach einem Schütteln, der Sensoreinheit eingehalten werden muss. Das oder jedes Kriterium ist in einer rechnerauswertbaren Form in einem Datenspeicher abgespeichert, auf den das Auswerte-Programm wenigstens zeitweise Lesezugriff hat.

Dank des Auswerte-Programms lässt sich automatisch entscheiden, ob die Sensoreinheit korrekt geschüttelt wurde oder aber zu schwach / zu stark und / oder zu kurz / zu lang geschüttelt wurde. Dadurch lässt sich in vielen Fällen entscheiden, ob ein Untersuchungsergebnis der Sensoreinheit gültig ist oder nicht.

In vielen Fällen ist es erforderlich, die Sensoreinheit zunächst auf eine bestimmte Weise zu schütteln und anschließend die Sensoreinheit für eine bestimmte Zeitdauer ruhig zu halten. Durch das Schütteln wird die Probe mit dem oder jedem Reagens des Sensors vermischt. In dieser Ruhephase findet die chemische Reaktion statt und / oder kommt in der Pause zu einem Ende. Indem das Auswerte-Programm automatisch den tatsächlichen zeitlichen Verlauf der Bewegung im Raum auswertet, vermag das Auswerte-Programm automatisch zu entscheiden, ob eine Ruhepause von der erforderlichen Dauer eingehalten worden ist oder nicht. Optional umfasst die Überwachungseinheit zusätzlich eine Uhr, welche die Dauer der Ruhephase zu messen vermag. In der Regel umfasst ein tragbarer Rechner eine hierfür geeignete Systemuhr.

In einer bereits beschriebenen Anwendung lassen sich Ergebnisse des Auswerte-Programms dafür verwenden, um im Nachherein festzustellen, ob die Sensoreinheit korrekt bewegt wurde oder nicht. In einer anderen Ausgestaltung lassen sich Ergebnisse des Auswerte-Programms dafür verwenden, um bereits beim Einsatz zu entscheiden, ob die Sensoreinheit bereits ausreichend bewegt worden ist und / oder ausreichend lange ruhig gehalten worden ist oder noch mehr bewegt und / oder noch länger ruhig gehalten werden muss.

Bevorzugt vermag das Auswerte-Programm eine Ausgabeeinheit anzusteuern. Die angesteuerte Ausgabeeinheit vermag eine Information in einer von einem Menschen wahrnehmbaren Form auszugeben, insbesondere visuell auszugeben. Die Ausgabeeinheit kann zu der Überwachungseinheit gehören oder zu einem räumlich entfernten tragbaren oder stationären Rechner. Das Auswerte-Programm vermag das Signal des Bewegungs-Sensors auszuwerten und abhängig von dieser Auswertung eine Nachricht zu erzeugen. In einer Ausgestaltung umfasst diese Nachricht die Information, ob die Sensoreinheit ein gültiges Ergebnis zu liefern vermag oder nicht. In einer anderen Ausgestaltung spezifiziert diese Nachricht, wie lange und / oder wie stark die Sensoreinheit noch bewegt werden muss, um die Probe zuverlässig zu untersuchen, und / oder legt fest, dass die Sensoreinheit jetzt nicht mehr bewegt werden soll, und / oder spezifiziert, wie lange sie noch ruhig gehalten werden muss. Das Auswerte-Programm löst den Schritt aus, dass diese Nachricht auf der Ausgabeeinheit ausgegeben wird.

In manchen Fällen führt diese Ausgestaltung dazu, dass die Sensoreinheit aufgrund der ausgegebenen Nachricht doch noch korrekt bewegt und / oder ruhig gehalten wird, obwohl sie vor Ausgabe der Nachricht nicht korrekt bewegt, insbesondere nicht lange oder nicht stark genug bewegt, oder nicht lange genug ruhig gehalten worden ist. Daher vermag diese Ausgestaltung in manchen Fällen den Verbrauch an Sensoreinheiten zu reduzieren.

In einer Ausgestaltung ist das Auswerte-Programm auf der Überwachungseinheit selber installiert und wird von der Überwachungseinheit ausgeführt. Beim Ausführen steuert das Auswerte-Programm Bestandteile der Überwachungseinheit an. Falls als Überwachungseinheit ein tragbarer Rechner verwendet wird, so umfasst dieser tragbare Rechner in vielen Fällen bereits die erforderlichen Prozessoren und Datenspeicher, um das Auswerte-Programm auszuführen. Das Auswerte-Programm kann auf einem üblichen Betriebssystem ablauffähig sein. Die Ausgestaltung, dass das Auswerte-Programm auf der Überwachungseinheit selber installiert ist, macht es einfacher, ein Signal des oder eines Überwachungs-Sensors dem Auswerte-Programm zur Verfügung zu stellen. Erspart wird eine drahtlose oder kabelgebundene Datenverbindung zwischen der Überwachungseinheit und einem räumlich entfernten Rechner, auf dem das Auswerte-Programm installiert ist.

In einer anderen Ausgestaltung wird eine spezielle Überwachungseinheit verwendet, welche den oder jeden Überwachungs-Sensor sowie optional eine Lichtquelle umfasst. Diese Überwachungseinheit steht wenigstens zeitweise in einer Datenverbindung mit einem separaten, insbesondere räumlich entfernten Rechner. Das Auswerte-Programm ist auf diesem Rechner installiert, empfängt das Signal des Bewegungs-Sensors und steuert bevorzugt bei der Ausführung Bestandteile dieses Rechners an. Die Datenverbindung kann eine drahtlose oder eine kabelgebundene Verbindung sein und nutzt eine Kommunikationseinheit der Übertragungseinheit und eine Kommunikationseinheit des räumlich entfernten Rechners. Der Rechner kann ein tragbarer Rechner, insbesondere ein Smartphone oder ein Tablet, oder ein stationärer Rechner sein.

Das Merkmal, dass eine spezielle Überwachungseinheit mit dem Bewegungs-Sensor und ein von der Überwachungseinheit getrennter Rechner mit dem Auswerte-Programm verwendet werden, erspart die Notwendigkeit, dass das Auswerte-Programm auf dieser Überwachungseinheit ablauffähig ist. Die Überwachungseinheit benötigt dann weder einen Prozessor, der das Auswerte-Programm auszuführen vermag, noch einen Datenspeicher noch eine Spannungsversorgungseinheit für den Prozessor. Dadurch verbraucht die Überwachungseinheit weniger elektrische Energie, nämlich nur die elektrische Energie, die zum Betrieb der Überwachungs-Sensoren und optional der Lichtquelle erforderlich ist. In vielen Fällen lässt sich eine spezielle Überwachungseinheit daher relativ leicht ausführen (geringeres Gewicht). Der separate Rechner braucht nicht mit der Sensoreinheit verbunden und optional geschüttelt zu werden, sondern nur die Überwachungseinheit. Der separate Rechner brauchen nicht den Bewegungen der Sensoreinheit standhalten zu können. Wenn eine spezielle Überwachungseinheit verwendet wird, lässt diese Überwachungseinheit sich in vielen Fällen leichter hinsichtlich ihrer Abmessungen und ihrer Geometrie an die Sensoreinheit anpassen. Diese leichtere Anpassbarkeit ist insbesondere dann von Vorteil, wenn eine bereits vorhandene Sensoreinheit, beispielsweise eine bestimmte Baureihe, weiterverwendet werden soll. In vielen Fällen braucht die Sensoreinheit nicht abgewandelt zu werden, und die spezielle Überwachungseinheit wird an die bereits vorhandene Sensoreinheit angepasst.

In einer Ausgestaltung umfasst der oder ein Überwachungs-Sensor ein Thermometer. Das Thermometer vermag die Temperatur in der Umgebung der Überwachungseinheit zu messen. Wenn die Sensoreinheit mit der Überwachungseinheit verbunden ist, misst dieses Thermometer auch die Temperatur in der Umgebung der Sensoreinheit. Diese Umgebungstemperatur kann die oder mindestens eine chemische Reaktion, die in der Sensoreinheit abläuft, und daher auch die Dauer und / oder Stärke der erforderlichen Bewegung beeinflussen. Bei manchen Umgebungstemperaturen kann es unmöglich sein, dass die Sensoreinheit gültige Ergebnisse liefert.

In einer Ausgestaltung umfasst die Überwachungseinheit eine Ausgabeeinheit, insbesondere einen Bildschirm. Auf dieser Ausgabeeinheit vermag die Überwachungseinheit Nachrichten in einer von einem Menschen wahrnehmbaren Form auszugeben. In einer Ausgestaltung spezifiziert diese Nachricht eine geforderte Behandlung der Sensoreinheit. Beispielsweise spezifiziert die Nachricht, wie lange die Sensoreinheit zu schütteln und / oder anschließend ruhig zu halten ist. Die Nachricht kann auch ein Ergebnis der Sensoreinheit beschreiben.

Die Ausgestaltung mit dem Thermometer und die Ausgestaltung mit der Ausgabeeinheit lassen sich kombinieren. Gemäß dieser Kombination vermag die Überwachungseinheit eine Nachricht zu erzeugen, welche von der Umgebungstemperatur abhängt, die das Thermometer gemessen hat.

Die Dauer des Schüttelns kann von der Umgebungstemperatur abhängen, insbesondere dergestalt, dass die erforderliche Dauer des Schüttelns umso kürzer ist, je höher die Umgebungstemperatur ist. Möglich ist, dass auf der Ausgabeeinheit eine Nachricht ausgegeben wird, jetzt das Schütteln zu beenden. In manchen Fällen vermeidet die Ausgabe der Nachricht somit, dass die Sensoreinheit länger als nötig geschüttelt wird. Dies spart Zeit ein. In einer Alternative legt die Nachricht fest, ob bei der gemessenen Umgebungstemperatur die Sensoreinheit überhaupt ein gültiges Ergebnis zu erzielen vermag oder nicht. Die Überwachungseinheit vermag die erzeugte Nachricht auf der Ausgabeeinheit auszugeben.

Erfindungsgemäß lässt sich die Sensoreinheit mit der Überwachungseinheit mechanisch verbinden. In einer Ausgestaltung umfasst die Überwachungseinheit einen Grundkörper sowie eine Halteplatte. Die Halteplatte ist mechanisch mit dem Grundkörper verbunden. Die Sensoreinheit lässt sich auf die Halteplatte stellen. Die Sensoreinheit befindet sich dann neben dem Grundkörper. Die Halteplatte trägt die Sensoreinheit. In vielen Fällen lässt sich dank dieser Ausgestaltung die Sensoreinheit besonders sicher mit einer derartig ausgestalteten Überwachungseinheit verbinden, und die Gefahr wird reduziert, dass die Sensoreinheit sich relativ zur Überwachungseinheit bewegen kann, wenn sie mit der Überwachungseinheit verbunden ist und beide bewegt werden. Bevorzugt lässt die Sensoreinheit sich lösbar mit dem Grundkörper verbinden, insbesondere mittels einer Schnappverbindung oder Rastverbindung oder einer magnetischen Verbindung oder mindestens einem Haken.

Bevorzugt umfasst die Halteplatte einen umlaufenden Rand, der ein Becken begrenzt, in welches sich die Sensoreinheit stellen lässt. Gemäß dieser Realisierungsform wird die Sensoreinheit besonders sicher auf der Halteplatte gehalten.

Bevorzugt lässt die Halteplatte sich relativ zum Grundkörper verschwenken, insbesondere drehen, wenn die Überwachungseinheit nicht mit der Sensoreinheit verbunden ist. Diese Ausgestaltung ermöglicht es, die Überwachungseinheit in eine platzsparende Transportposition zu verbringen, in welcher die Halteplatte am Grundkörper anliegt. Wenn die Halteplatte von dem Grundkörper weg gedreht ist, lässt die Sensoreinheit sich auf die Halteplatte stellen. Möglich ist, dass die Überwachungseinheit automatisch eingeschaltet wird, wenn die Halteplatte vom Grundkörper weg bewegt wird, und ausgeschaltet wird, wenn die Halteplatte zum Grundkörper hin und in die Transportposition bewegt wird. Diese Ausgestaltung spart oft elektrische Energie ein, weil verhindert wird, dass die Überwachungseinheit in einer Ruhepause eingeschaltet bleibt.

In einer Ausgestaltung umfasst die Überwachungseinheit eine Ausgabeeinheit, insbesondere einen Bildschirm. Die Überwachungseinheit vermag auf dieser Ausgabeeinheit in einer von einem Menschen wahrnehmbaren Form eine Benutzungsanleitung für die Sensoreinheit anzugeben, insbesondere eine Anleitung, wie eine Probe in die Eingabeeinheit einzugeben ist und / oder auf welche Weise eine Mess-Anordnung bestehend aus der Überwachungseinheit und der mit der Überwachungseinheit verbundenen Sensoreinheit zu bewegen ist, damit ein Reagens des Sensors mit der Probe vermischt werden kann und der Indikator das Ergebnis der chemischen Reaktion anzeigen kann. Indem diese Benutzungsanleitung auf der Ausgabeeinheit ausgegeben wird, wird die Gefahr reduziert, dass die Sensoreinheit falsch benutzt wird. Beispielsweise wird eine Meldung ausgegeben, jetzt das Schütteln zu beginnen und / oder zu beenden.

In einer Ausgestaltung erzeugt die Überwachungseinheit die Benutzungsanleitung abhängig von einem Messergebnis des optionalen Thermometers, welches die Umgebungstemperatur in der Umgebung der Überwachungseinheit misst. Beispielsweise verwendet die Überwachungseinheit ein rechnerverfügbares Template (Muster) für die Benutzungsanleitung und setzt in dieses Template mindestens einen Parameter ein, der von der gemessenen Umgebungstemperatur abhängt. Dieser Parameter spezifiziert insbesondere, wie lange die Mess-Anordnung zu bewegen und / oder ruhigzuhalten ist. Auf diese Weise passt die Benutzungsanleitung zu der aktuellen Umgebungstemperatur.

In einer Ausgestaltung lässt die Überwachungseinheit sich wahlweise in einem vom zwei unterschiedlichen Modi betreiben, nämlich in einem Überwachungs-Modus oder in einem Dokumentations-Modus. Wenn die Überwachungseinheit im Überwachungs-Modus betrieben wird, so wird die Sensoreinheit nach der Eingabe der Probe mit der Überwachungseinheit verbunden, und die hierdurch entstandene Mess-Anordnung wird bewegt. Ein Bewegungs-Sensor der Überwachungseinheit misst die Bewegung der Mess-Anordnung und erzeugt ein Signal.

Wenn die Überwachungseinheit im Dokumentations-Modus betrieben wird, so wird die Verbindung zwischen der Sensoreinheit und der Überwachungseinheit hergestellt, wenn der oder mindestens ein Indikator das Ergebnis einer chemischen Reaktion visuell anzeigt oder anzuzeigen beginnt. Eine Kamera der Überwachungseinheit erzeugt mindestens ein Bild, welches den oder mindestens einen, bevorzugt jeden Indikator zeigt. Das oder jedes Bild vom Indikator lässt sich abspeichern, und dadurch lässt sich das Ergebnis der Untersuchung dokumentieren. In einer Ausgestaltung wird das oder jedes Bild vom Indikator manipulationssicher abgespeichert, und das Ergebnis der chemischen Reaktion lässt sich bei Bedarf gerichtsfest dokumentieren. Beim Betrieb im Dokumentations-Modus ist die Überwachungseinheit bevorzugt kürzer mit der Sensoreinheit verbunden. Dadurch lässt sich dieselbe Überwachungseinheit nacheinander mit einer größeren Anzahl von unterschiedlichen Sensoreinheiten verbinden, als wenn die Überwachungseinheit im Überwachungs-Modus betrieben wird. In derselben Zeitspanne lässt sich eine größere Anzahl von Proben untersuchen. Auch beim Betrieb im Überwachungs-Modus braucht die Sensoreinheit keinen elektrischen Verbraucher und daher auch keine Spannungsversorgungseinheit zu umfassen.

In einer Anwendung wird die erfindungsgemäße Anordnung dafür verwendet, um eine Speichelprobe oder einen Abstrich aus der Nase oder einem Ohr oder dem Gaumen eines Menschen oder eines sonstigen Lebewesens auf mindestens eine Substanz zu untersuchen. Diese Substanz ist insbesondere eine Droge oder ein Medikament. In einer anderen Anwendung wird die erfindungsgemäße Anordnung dafür verwendet, um eine Probe, die aus der Nase oder dem Ohr oder dem Mund eines Menschen oder sonstigen Lebewesens entnommen worden ist, auf Mikroben oder Viren zu untersuchen. Möglich ist auch, dass die zu untersuchende Probe von einer Oberfläche eines Gegenstands entnommen wurde und festgestellt werden soll, ob auf dieser Oberfläche eine Droge oder ein Medikament lag.

Im Folgenden wird die Erfindung anhand eines Ausführungsbeispiels beschrieben. Hierbei zeigt
- Figur 1: in zwei perspektivischen Darstellungen links eine Sammeleinheit und rechts eine stationäre Basisstation, in welcher die Sammeleinheit geschüttelt und eine Speichelprobe ausgewertet wird;
- Figur 2: in einer perspektivischen Darstellung die Sensoreinheit und in einer Frontdarstellung die Anzeigefläche der Sensoreinheit;
- Figur 3: schematisch in einer Seitenansicht die erfindungsgemäße Anordnung mit Sensoreinheit, Überwachungseinheit und Auswerteeinheit, wobei die Sensoreinheit neben der Überwachungseinheit positioniert ist;
- Figur 4: die Sensoreinheit und die Überwachungseinheit von Figur 3 mit der Sensoreinheit hinter der Überwachungseinheit;
- Figur 5: die Anordnung von Figur 4 in einer Hand eines Benutzers;
- Figur 6: eine abweichende Ausgestaltung einer erfindungsgemäßen Überwachungseinheit, die einen eigenen Bildschirm aufweist;
- Figur 7: eine weitere Ausgestaltung, bei der ein Smartphone zusätzlich als Überwachungseinheit fungiert und mittels eines Adapters mit der Sensoreinheit verbunden ist.

Figur 1 zeigt ein aus dem Stand der Technik bekanntes Gerät mit einer Sammeleinheit 20 und einer stationären Basisstation 30. Ein solches Gerät ist unter der Bezeichnung "Dräger DrugTest 5000 System" bekannt geworden. Die Sammeleinheit 20 heißt "Test Kit" und die Basisstation 30 "Analyzer". Selbstverständlich wird die Sammeleinheit 20 nur zur Untersuchung einer einzigen Probe auf Drogen oder Medikamente verwendet.

Die Sammeleinheit 20 umfasst einen Probensammler 21, eine Kappe 24 für den Probensammler 21, eine Aufnahme 22 und ein Gehäuse 23. Ein Proband gibt eine Speichelprobe ab, wobei der Proband den Probensammler 21 in den Mund nimmt und bewegt. Die abgegebene Speichelprobe fließt in eine Kammer im Inneren des Gehäuses 23. Der Probensammler 21 ist über eine Aufnahmeeinheit 22 fest oder lösbar mit dem Gehäuse 23 verbunden.

Ein Prüfer schiebt die Sammeleinheit 20 mit der abgegebenen Speichelprobe durch die verschließbare Öffnung 31 hindurch in das Innere der Basisstation 30. Ein nicht gezeigter Sensor befindet sich im Inneren der Basisstation 30 und untersucht die Speichelprobe auf unterschiedliche Drogen und / oder Medikamente. Anschließend zeigt die Anzeigeeinheit 36 das Ergebnis an. Die Basisstation 30 umfasst mehrere Bedienelemente 32 in Form von Knöpfen.

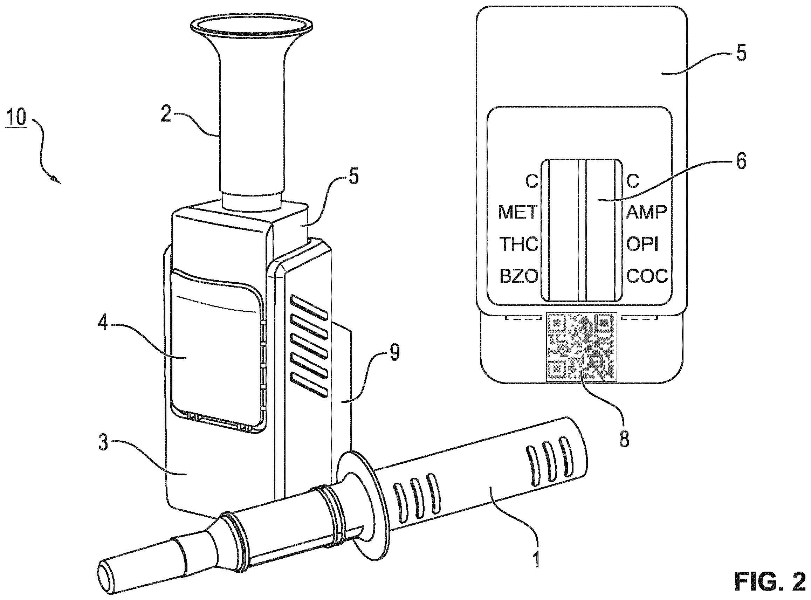

Figur 2 zeigt links in einer perspektivischen Darstellung eine beispielhafte tragbare Sensoreinheit, welche ein Bestandteil des Ausführungsbeispiels der erfindungsgemäßen Anordnung ist, und rechts in einer vergrößerten Frontdarstellung eine Anzeigefläche dieser Sensoreinheit. Zwei solche Sensoreinheiten sind unter den Bezeichnungen "Dräger DrugCheck 3000" und "Dräger Antigen Test SARS-CoV-2" bekannt geworden. Eine solche Sensoreinheit wird auch in DE 10 2014 001 386 A1 beschrieben. Die gesamte Sensoreinheit wird für eine einzige Probe auf Drogen und / oder Medikamente verwendet. Für die Erfindung lässt sich auch eine andere tragbare Sensoreinheit verwenden.

Anmerkung: Dieselbe Substanz kann in manchen Anwendungen ein Medikament sein, welches die Gesundheit eines Menschen verbessert, und in anderen Fällen eine Droge, die den Menschen schädigt und Abhängigkeit erzeugt. Daher wird im Folgenden der allgemeine Begriff "Substanz" verwendet. Die Sensoreinheit 10 vermag mindestens eine Substanz nachzuweisen, wobei die oder jede Substanz insbesondere eine Droge oder ein Medikament ist.

Die Sensoreinheit 10 von Figur 2 umfasst
- eine Kassette 5 mit einer Messkammer und einer Ampulle aus einem zerbrechlichen Material, z.B. aus Glas, wobei in der Ampulle mindestens ein Reagens untergebracht ist,
- mehrere Papierstreifen in der Kassette 5,
- eine durchsichtige Anzeigefläche 6 an der Kassette 5,
- eine eindeutige Kennung 8 in Form eines QR-Codes, wobei die Kennung 8 diese Sensoreinheit 10 von jeder anderen Sensoreinheit unterscheidet,
- ein Gehäuse 3, das die Kassette 5 umgibt,
- eine Aufnahmetasche 9 am Gehäuse 3,
- eine Abdeckung 4 für die Anzeigefläche 6, wobei die Abdeckung 4 zum Gehäuse 3 gehört,
- eine röhrenförmige Aufnahmeeinheit 2, die fest mit der Kassette 5 verbunden ist, und
- einen hohlen Probensammler 1, der sich in die Aufnahmeeinheit 2 einsetzen lässt.

Auf jeden Papierstreifen in der Kassette 5 sind Antikörper aufgebracht. Bevorzugt werden Proteine (Eiweiße) als Antikörper verwendet, wobei diese Proteine in Wirbeltieren gebildet werden, und zwar indem Körperzellen der Wirbeltiere Reaktionsprodukte erzeugen. Die Körperzellen erzeugen diese Proteine, indem sie auf bestimmte Stoffe reagieren, welche den Wirbeltieren verabreicht wurden. Diese verabreichten Stoffe werden oft als Antigene bezeichnet.

Im Ausführungsbeispiel werden diese Antikörper auf die Papierstreifen aufgebracht und fungieren als jeweils ein Reagens pro Papierstreifen. Jedes Reagens reagiert chemisch mit einer Substanz, die in der Speichelprobe enthalten sein kann. Jeder Papierstreifen ist also mindestens einer Substanz zugeordnet. Der Verlauf der chemischen Reaktion auf dem Papierstreifen hängt davon ab, ob diese Substanz tatsächlich in der Speichelprobe enthalten ist oder nicht, und in einer Ausgestaltung auch von der Konzentration dieser Substanz in der Speichelprobe. Nach Abschluss der chemischen Reaktion hat der Papierstreifen eine Farbe angenommen, wobei diese Farbe davon abhängt, ob die zugeordnete Substanz in der Speichelprobe enthalten ist oder nicht. Wenigstens jeweils ein Teil jedes Papierstreifens befindet sich hinter der Anzeigefläche 6, sodass auf oder hinter der Anzeigefläche 6 das jeweilige Ergebnis jeder chemischen Reaktion sichtbar ist. Jeder Papierstreifen fungiert also als ein Indikator für die zugeordnete Substanz.

Die Sensoreinheit 10 vermag im Ausführungsbeispiel automatisch bis zu sechs verschiedene Substanzen in einer einzigen Speichelprobe eines Probanden zu erkennen. Im Ausführungsbeispiel sind dies Metaamphetamine (MET), Cannabis (THC), Benzodiazepine (BZO), Amphetamin (AMP), Opiate (OPI) und Kokain (COC). Die Sensoreinheit 10 hat keinen elektrischen Verbraucher und benötigt keine elektrische Energie. Das Reagens in der Ampulle und das Reagens auf einem Papierstreifen bilden zusammen ein Reagens für die zugeordnete Substanz. Auf der Anzeigefläche 6 sind daher sechs Indikatoren in Form von sechs Papierstreifen sichtbar.

Vor einem Einsatz ist der Probensammler 1 in die Aufnahmetasche 9 eingesetzt. Die gesamte Sensoreinheit 10 ist fluiddicht und bevorzugt mit einem Unterdruck in einen nicht gezeigten Beutel aus einer Metallfolie eingeschweißt.

Um einen Probanden auf die sechs möglichen Substanzen zu untersuchen, wird die Sensoreinheit 10 aus dem Beutel entnommen. Der Proband gibt eine Speichelprobe in den Probensammler 1 ein. Hierbei nimmt er das dickere Teil des Probensammlers 1, das in Figur 2 rechts gezeigt wird, in die Hand und steckt das dünnere Ende in den Mund und bewegt den Probensammler 1 im Mund. Ein farbiger Ring am dünneren Ende des Probensammlers 1 verschwindet, wenn der Probensammler 1 genügend Speichel aufgenommen hat.

In einer nicht gezeigten Abwandlung steckt der Proband das dünnere Ende nacheinander in seine beiden Nasenflügel und bewegt den Probensammler in der Nase. Diese Abwandlung wird insbesondere dafür benutzt, um den Probanden auf ein Virus, insbesondere auf das Corona-Virus, zu untersuchen.

Die folgende Beschreibung bezieht sich auf eine Probe in Form einer flüssigen Speichelprobe. Sie gilt entsprechend auch für andere Proben. Nachdem der Proband die Speichelprobe abgegeben hat, wird der Probensammler 1 mit der aufgenommenen Speichelprobe von oben in die Aufnahmeeinheit 2 eingesetzt, wobei das dünne Ende zur Kassette 5 hin zeigt. Beim Einsetzen zerbricht die Ampulle in der Kassette 5. Der Speichel fließt aus dem Probensammler 1 in die Messkammer in der Kassette 5. Das Reagens fließt aus der zerbrochenen Ampulle in die Messkammer.

Ein Prüfer schüttelt die gesamte Sensoreinheit 10. In der Messkammer wird durch das Schütteln eine Mischung aus der Speichelprobe und dem Reagens aus der Ampulle gebildet. Anschließend hält der Prüfer die Sensoreinheit 10 ruhig. Durch das Schütteln und die nachfolgende Ruhepause kommt die chemische Reaktion zwischen der Speichelprobe und dem Reagens in der Messkammer zu einem Abschluss. Der Prüfer ist ein Mensch und kann der Proband sein oder ein anderer Mensch.

Nach dieser Ruhepause bricht der Prüfer die Abdeckung 4 von dem Gehäuse 3 ab und drückt die Kassette 5 von oben in das Gehäuse 3 hinein. Hierdurch kommt das Gemisch aus der Speichelprobe und dem Reagens in Kontakt mit den sechs Indikator-Papierstreifen. Jeder Indikator-Papierstreifen verfärbt sich abhängig davon, ob die zugeordnete Substanz in der Speichelprobe enthalten ist oder nicht. Nach einiger Zeit werden die Untersuchungsergebnisse auf der Anzeigefläche 6 sichtbar.

In dem gezeigten Ausführungsbeispiel erscheinen dann, wenn die Kassette 5 die Speichelprobe untersuchen konnte, neben den beiden C (Kontrollbereich) der Anzeigefläche 6 jeweils ein waagrechter Strich. Falls diese beiden Striche nicht erscheinen, liefert die Untersuchung kein gültiges verwendbares Untersuchungsergebnis. Ein möglicher Grund dafür, dass diese beiden Striche nicht erscheinen, ist der folgende: Die Kassette 5 wurde nicht weit genug oder auf andere Weise nicht richtig in das Gehäuse 3 hineingedrückt.

Falls in der Speichelprobe keine der sechs Substanzen in einer Konzentration oberhalb der jeweiligen Nachweisgrenze vorhanden ist, erscheinen in den beiden Spalten der Anzeigefläche 6 jeweils ein waagerechter Strich neben jedem Kürzel für eine Substanz, insgesamt also sechs weitere Striche. Fehlt neben einem Kürzel ein waagerechter Strich, so wurde die entsprechende Substanz in der Speichelprobe entdeckt.

Damit die Sensoreinheit 10 gemäß Figur 2 zuverlässige Ergebnisse liefert, muss sie nach der Abgabe der Probe in einer bestimmten Weise bewegt werden. Ansonsten wird kein gültiges Untersuchungsergebnis bereitgestellt. Das System gemäß Figur 1 erfordert die stationäre Überwachungseinheit 30, welche einen Anschluss an ein stationäres Spannungsversorgungsnetz oder eine eigene Spannungsversorgungseinheit benötigt und einen gewissen Platz einnimmt. Im Ausführungsbeispiel ist keine stationäre Basisstation erforderlich.

Figur 3 zeigt in einer Seitenansicht schematisch eine erste Ausgestaltung der erfindungsgemäßen Anordnung, welche keine stationäre Basisstation erfordert und trotzdem die Gefahr verringert, dass die Sensoreinheit 10 nach der Probenabgabe falsch bewegt wird. Gleiche Bezugszeichen haben die gleiche Bedeutung wie in Figur 2.

Die erfindungsgemäßen Anordnung gemäß Figur 3 umfasst
- die Sensoreinheit 10, die im Ausführungsbeispiel genauso wie die Sensoreinheit 10 von Figur 2 ausgestaltet ist,
- eine mobile Überwachungseinheit 40 und
- eine signalverarbeitende Auswerteeinheit 70.

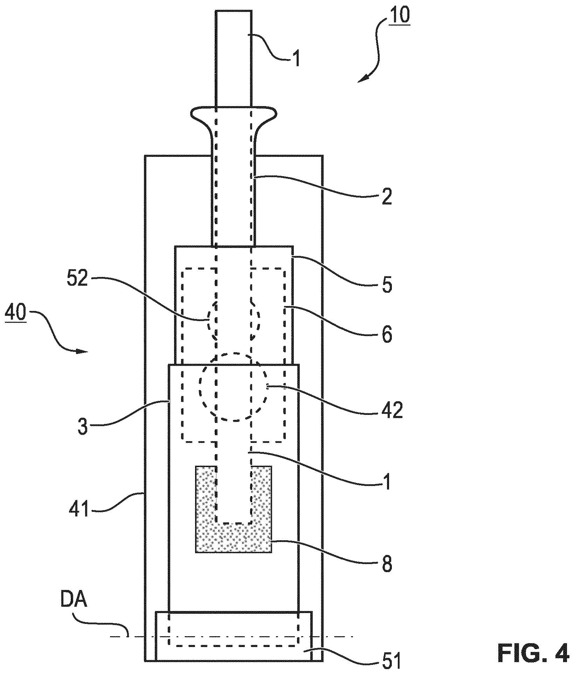

Figur 4 zeigt die Sensoreinheit und die Überwachungseinheit von Figur 3 mit der Sensoreinheit 10 hinter der Überwachungseinheit 40. Die Sensoreinheit 10 ist in Figur 3 rechts neben und in Figur 4 vor der Überwachungseinheit 40 angeordnet.

Die mobile Überwachungseinheit 40 hat im Ausführungsbeispiel die Form eines L und umfasst
- ein Gehäuse 41,
- eine Kamera 42, die am Gehäuse 41 montiert ist,
- eine optionale Lichtquelle 52, die ebenfalls am Gehäuse 41 montiert ist,
- einen Beschleunigungs-Sensor 43,
- optional ein Thermometer 49,
- eine basisseitige Kommunikationseinheit 45,
- ein Steuergerät 46 und
- eine eigene Spannungsversorgungseinheit 47.

Das Gehäuse 41 umschließt einen Grundkörper der Überwachungseinheit 40. An diesen Grundkörper ist eine verschwenkbare rechteckige Halteplatte 51 befestigt, welche eine Sensoreinheit 10 zu tragen vermag. Bevorzugt weist die Halteplatte 51 einen umlaufenden hochstehenden Rand auf, sodass die Halteplatte 51 ein Becken ausbildet, in welches sich die Sensoreinheit 10 stellen lässt. Diese Halteplatte 51 lässt sich nach oben um die Drehachse DA klappen, um die Überwachungseinheit 40 zu transportieren, wenn keine Sensoreinheit in dem Becken steht.

Die Überwachungseinheit 40 kann auch die Form einer Röhre oder Säule haben, die nach oben offen ist.

Der Beschleunigungs-Sensor 43, das Steuergerät 46, das Thermometer 49 und die Spannungsversorgungseinheit 47 sind im Inneren des Gehäuses 41 angeordnet. Bevorzugt ist auch die basisseitige Kommunikationseinheit 45 im Inneren des Gehäuses 41 angeordnet.

Die Auswerteeinheit 70 ist im Ausführungsbeispiel auf einem Smartphone realisiert. Sie kann auch auf einem sonstigen tragbaren oder stationären Rechner realisiert sein. Die Auswerteeinheit 70 umfasst
- ein Gehäuse 75,
- Auswerte-Software 71, die auf einem Rechner ausführbar ist, z.B. eine App,
- einen Datenspeicher 76,
- einen Bildschirm 77,
- eine auswerteseitige Kommunikationseinheit 72,
- eine Uhr 44,
- ein signalverarbeitendes Steuergerät 73 und
- eine eigene Spannungsversorgungseinheit 74 und / oder eine Verbindung zu einem stationären Spannungsversorgungsnetz.

Die Auswerteeinheit 70 kann ein handelsübliches Smartphone oder ein sonstiger tragbarer oder auch stationärer Rechner sein. Speziell ist die Auswerte-Software 71, welche auf einem Prozessor der Auswerteeinheit 70 abläuft und bevorzugt die Form einer App für einen Rechner aufweist.

Das Steuergerät 73, die Uhr 44 und die Spannungsversorgungseinheit 74 sind im Inneren des Gehäuses 75 angeordnet, die Kommunikationseinheit 72 sowie der Bildschirm 57 außen am oder im Gehäuse 75. Die Auswerteeinheit 70 vermag auf dem Bildschirm 77 Meldungen in einer von einem Menschen wahrnehmbaren Form visuell auszugeben und dann, wenn der Bildschirm 77 als Touchscreen ausgestaltet ist, Benutzereingaben zu erfassen. Optional vermag die Auswerteeinheit 70 auch akustische Mitteilungen auszugeben und / oder eine Vibration der Auswerteeinheit 70 zu bewirken, also Meldungen in einer haptisch Form auszugeben.

Wie bereits erwähnt, benötigt die Sensoreinheit 10 keine Versorgung mit elektrischer Energie. Die Überwachungseinheit 40 hat eine eigene Spannungsversorgungseinheit 47. Daher sind die Sensoreinheit 10 und die Überwachungseinheit 40 für einen mobilen Einsatz geeignet, beispielsweise für Einsatzkräfte, die Teilnehmer am Straßenverkehr auf vorgegebene Substanzen kontrollieren. Die Auswerteeinheit 70 kann von der Sensoreinheit 10 und der Überwachungseinheit 40 räumlich entfernt sein.

Die Sensoreinheit 10 lässt sich lösbar mit der Überwachungseinheit 40 verbinden. Im Ausführungsbeispiel lässt die Sensoreinheit 10 sich auf die heruntergeklappte Halteplatte 51 stellen. Die lösbare Verbindung lässt sich beispielsweise mit Klammern, einer Rastverbindung, einer Schnappverbindung, einer Presspassung und / oder durch Magnete herstellen. Falls die Überwachungseinheit 40 die Form einer oben offenen Röhre oder Säule hat, lässt die Sensoreinheit 10 sich von oben einschieben. Auch bei dieser Ausgestaltung wird die Sensoreinheit 10 bevorzugt zusätzlich mit Klammern, einer Rastverbindung, einer Schnappverbindung, einer Presspassung, Haken und / oder durch Magnete lösbar an der Überwachungseinheit 40 befestigt

Nachdem die lösbare Verbindung hergestellt ist, vermag die Sensoreinheit 10 idealerweise keine Bewegungen relativ zur Überwachungseinheit 40 auszuführen. Im Folgenden wird die Anordnung, die aus der Überwachungseinheit 40 und der Sensoreinheit 10 besteht, wobei die Sensoreinheit 10 mit der Überwachungseinheit 40 verbunden ist, als "Mess-Anordnung 10, 40" bezeichnet.

Die Sensoreinheit 10 wird für eine einzige Probeneingabe verwendet, während die Überwachungseinheit 40 sich nacheinander für mehrere Probeneingaben verwenden lässt, und zwar mit jeweils einer Sensoreinheit 10. Möglich ist, dieselbe Überwachungseinheit 40 nacheinander mit unterschiedlichen Sensoreinheiten 10, beispielsweise für unterschiedliche Substanzen, zu verwenden.

In einer Ausgestaltung (nicht gezeigt) ist die Abdeckung 4 durchsichtig. In einer anderen Ausgestaltung, die in Figur 2 gezeigt wird, ist die Abdeckung 4 nicht durchsichtig. Damit die Anzeigefläche 6 sichtbar wird, entfernt der Prüfer die Abdeckung 4 von dem Gehäuse 2. Bei Bedarf schiebt der Prüfer die Sensoreinheit 10 relativ zur Überwachungseinheit 40 leicht nach oben. Nachdem die Abdeckung 4 entfernt ist, drückt der Prüfer die Kassette 5 so wie oben beschrieben nach unten in das Gehäuse 3 ein, und automatisch wird der Schritt ausgelöst, dass die sechs Indikator-Papierstreifen mit dem Gemisch aus Speichelprobe und Reagens in Kontakt kommen. Auf der Anzeigefläche 6 der Sensoreinheit 10 erscheinen so wie oben beschrieben die Ergebnisse der Auswertung.

Der Beschleunigungs-Sensor 43 misst ein Maß für den zeitlichen Verlauf der Beschleunigung, den die Überwachungseinheit 40 in einem ortsfesten dreidimensionalen Koordinatensystem ausführt. Zu jedem Abtast-Zeitpunkt wird jeweils ein Messwert mit drei Koordinaten erzeugt. Dadurch erzeugt der Beschleunigungs-Sensor 43 in Verbindung mit einer optionalen Messwertaufbereitung ein Bewegungs-Signal. Falls die Sensoreinheit 10 mit der Überwachungseinheit 40 verbunden ist, enthält das Bewegungs-Signal des Beschleunigungs-Sensors 43 die Informationen, in welche Richtungen, wie stark und wie lange die Sensoreinheit 10 geschüttelt wurde. Außerdem enthält das Bewegungs-Signal die Information, wie lange die Sensoreinheit 10 anschließend ruhig gehalten wurde.

Das Thermometer 49 steht in einem thermischen Kontakt mit der Umgebung und vermag die Umgebungstemperatur zu messen. In einer Ausgestaltung wird die gemessene Umgebungstemperatur für die Entscheidung verwendet, ob die Untersuchungsergebnisse der Sensoreinheit 10 gültig sein können oder bei dieser Umgebungstemperatur grundsätzlich ungültig sind. In einer anderen Ausgestaltung empfängt das Steuergerät 46 Messwerte vom Thermometer 49 und steuert eine Heizung und / oder eine Kühleinheit an, um die Temperatur in der Nähe der Kassette 5 zu vergrößern bzw. zu verkleinern. In einer dritten Ausgestaltung wird eine Nachricht mit der gemessene Umgebungstemperatur an die Auswerteeinheit 70 übermittelt, und die Verwendung dieser Nachricht wird weiter unten beschrieben.

Wenn die Verbindung zwischen der Sensoreinheit 10 und der Überwachungseinheit 40 hergestellt ist, vermag die optionale Lichtquelle 52 die Anzeigefläche 6 zu beleuchten. Das Licht, welches die Lichtquelle 52 aussendet, kann Licht im sichtbaren Bereich und / oder im Infrarotbereich und / oder im ultravioletten Bereich sein. In einer Realisierungsform vermag die Lichtquelle 52 die Wellenlänge des ausgesandten Lichts zu verändern.

Die Kamera 42 ist bei hergestellter Verbindung auf die Anzeigefläche 6 gerichtet und vermag daher Aufnahmen von den angezeigten Untersuchungsergebnissen aufzunehmen. Diese Aufnahmen zeigen insbesondere den QR-Code 8 und die oben beschriebenen bis zu sechs waagerechten Striche neben den sechs Kürzeln für sechs mögliche Substanzen auf der Anzeigefläche 6. Bevorzugt zeigen die Aufnahmen außerdem, ob ein gültiges Untersuchungsergebnis erzielt wurde (zwei waagerechte Striche neben dem C) oder nicht.

In einer Ausgestaltung fokussiert die Kamera 42 sich automatisch auf die Anzeigefläche 6. In einer anderen Ausgestaltung kann ein Benutzer den Fokus oder die Schärfeebene der Kamera 42 manuell anpassen. Beispielsweise ist eine entsprechende Betätigungseinheit 53 zum manuellen Fokussieren, insbesondere ein Drehrad, in das Gehäuse 41 eingelassen. Oder eine entsprechende Betätigungseinheit und eine Software-Anwendung zum manuellen Fokussieren sind auf der Überwachungseinheit 70 installiert. Möglich ist auch, dass eine Software-Anwendung auf der Auswerteeinheit 70 einen Benutzer dabei unterstützt, mithilfe der Betätigungseinheit 53 die Kamera 42 auf die Anzeigefläche 6 zu fokussieren.

Zwischen den Kommunikationseinheiten 45 und 72 lässt sich eine drahtlose Kommunikationsverbindung herstellen, beispielsweise per Bluetooth Low Energy oder einem Mobilfunk-Standard. Bevorzugt fragt die Auswerteeinheit 70 Untersuchungsergebnisse von der Überwachungseinheit 40 ab, und die Überwachungseinheit 40 übermittelt die angefragten Untersuchungsergebnisse an die Auswerteeinheit 70. Die Auswerte-Software 71 vermag übermittelte Untersuchungsergebnisse automatisch auszuwerten.

Wie bereits dargelegt, umfasst die Auswerteeinheit 70 einen Bildschirm 77. In einer bevorzugten Ausgestaltung erzeugt das Steuergerät 73 der Auswerteeinheit 70 automatisch eine Benutzungsanleitung für die Mess-Anordnung 10, 40 und veranlasst, dass diese Benutzungsanleitung auf dem Bildschirm 77 dargestellt wird. Diese Benutzungsanleitung spezifiziert insbesondere, wie stark und wie lange die Mess-Anordnung 10, 40 zu schütteln ist und wie lange sie anschließend ruhig gehalten werden soll. In einer Ausgestaltung hängt diese Benutzungsanleitung von der gemessenen Temperatur in der Umgebung der Sensoreinheit 10 ab. Wie oben bereits erwähnt, misst das Thermometer 49 diese Umgebungstemperatur. Das Steuergerät 73 erzeugt die Benutzungsanleitung also abhängig von der gemessenen Umgebungstemperatur. Bevorzugt verwendet das Steuergerät 73 hierfür ein Schema (ein Template) einer Benutzungsanleitung und setzt in dieses Schema mindestens einen Wert ein, der von der gemessenen Umgebungstemperatur abhängt, insbesondere eine Zeitdauer. Das Schema kann mindestens eine Berechnungsvorschrift umfassen, die von der Umgebungstemperatur abhängt.

In einer Ausgestaltung kann der Prüfer auswählen, ob er rasch oder mit höherer Zuverlässigkeit ein Untersuchungsergebnis wünscht. Das sicherere Untersuchungsergebnis erfordert mehr Zeit. Das Steuergerät 73 verwendet diese Vorgabe, um die Benutzungsanleitung entsprechend zu generieren.

Die Auswerte-Software 71 vergleicht die übermittelten Informationen über die gemessenen Beschleunigungen, welche auf die Sensoreinheit 10 einwirken, insbesondere Richtungen, Dauer und Stärke, und die gemessene Dauer der nachfolgenden Ruhepause mit einem vorgegebenen Soll-Verlauf des Schüttelns und einer vorgegebenen Soll-Dauer einer nachfolgenden Ruhepause. Die Mess-Anordnung 10, 40 wird geschüttelt, so dass die Speichelprobe mit dem Reagens vermischt wird, und dann ruhig gehalten, so dass die Indikator-Papierstreifen mit dem Gemisch chemisch reagieren. Die Auswerte-Software 71 entscheidet automatisch, ob die Sensoreinheit 10 korrekt geschüttelt wurde und anschließend korrekt ruhig gehalten wurde oder nicht. Hierfür verwendet die Auswerte-Software 71 das Bewegungs-Signal des Beschleunigungs-Sensors 43. Beispielsweise entscheidet die Auswerte-Software 71, ob der tatsächliche zeitliche Verlauf der Beschleunigung in einem vorgegebenen Toleranzbandes um den vorgegebenen Sollverlauf herum liegt oder nicht.

Bevorzugt wird anschließend dem Prüfer angezeigt, ob er die Mess-Anordnung 10, 40 korrekt geschüttelt und anschließend ruhig gehalten hat oder nicht. Optional wird dem Prüfer angezeigt, dass und wie lange er die Mess-Anordnung 10, 40 noch länger schütteln soll. Für die Anzeige, noch länger zu schütteln, verwendet die Auswerte-Software 71 das Bewegungs-Signal des Beschleunigungs-Sensors 43.

Die Sensoreinheit 10 zeigt die Untersuchungsergebnisse auf der Anzeigefläche 6 an, nachdem so wie oben beschrieben der Prüfer die Kassette 5 in das Gehäuse 3 hineingedrückt hat, wodurch die Auswertung der Speichelprobe begonnen wird, und nachdem eine vorgegebene Analyse-Zeitspanne verstrichen ist. Der Zeitpunkt, an dem die Auswertung der Speichelprobe begonnen wird, wird an die Auswerte-Software 71 übermittelt. Zu Beginn sowie nach Ablauf der Analyse-Zeitspanne veranlasst die Auswerte-Software 71, dass die Auswerteeinheit 70 jeweils einen entsprechenden Befehl an die Überwachungseinheit 40 übermittelt.

In einer Ausgestaltung erzeugt die Kamera 42 mit einer vorgegebenen Bildaufnahmefrequenz Bilder von der Anzeigefläche 6, nachdem die Auswertung einer Speichelprobe begonnen wurde. In einer anderen Ausgestaltung veranlasst die Überwachungseinheit 40 nach Erhalt eines entsprechenden Befehls von der Auswerteeinheit 70, dass die Kamera 42 mindestens ein Bild von der Anzeigefläche 6 erzeugt.

In einer Ausgestaltung erzeugt das Steuergerät 46 der Überwachungseinheit 40 oder das Steuergerät 73 der Überwachungseinheit 70 ein Auslösesignal und nachfolgend ein Beendigungssignal für die Kamera 42. Die Kamera 42 erzeugt mit der vorgegebenen Bildaufnahmefrequenz Bilder, nachdem sie das Auslösesignal erhalten hat und bis sie das Beendigungssignal erhält. Das Steuergerät 46, 73 erzeugt das Auslösesignal, nachdem durch Auswertung des Bewegungs-Signals vom Beschleunigungs-Sensor 43 festgestellt worden ist, dass die Mess-Anordnung 10, 40 bewegt worden ist und anschließend während einer vorgegebenen Zeitspanne nicht bewegt worden ist. Beispielsweise führt das Auswerte-Programm 71 diese Auswertung durch. Nach Ablauf dieser Zeitspanne mit der Ruhepause beginnen die Indikator-Papierstreifen 5 hinter der Anzeigefläche 6, sich sichtbar zu verändern. Die erzeugte Abfolge von Bildern zeigt diese Veränderung. Diese Veränderung korreliert in vielen Fällen mit der Konzentration einer Substanz in der Speichelprobe. Das Beendigungssignal erzeugt das Steuergerät 46, 73 beispielsweise, nachdem nach Erzeugung des Auslösesignals eine vorgegebene Zeitspanne verstrichen ist.

In einer bereits erwähnten Realisierungsform vermag die Lichtquelle 52 Licht in unterschiedlichen Wellenlängen auszustrahlen. Gemäß dieser Realisierungsform variiert die Lichtquelle 52 die Wellenlänge des ausgestrahlten Lichts, während die Kamera 42 Bilder von der Anzeigefläche 6 erzeugt. In vielen Fällen lässt sich durch diese variierten Wellenlängen noch besser die Veränderung der Anzeigefläche 6 und damit die Veränderung der Indikator-Papierstreifen 5 erkennen, auch durch automatische Bildauswertung.

In beiden Ausgestaltungen wird das oder jedes Bild von der Anzeigefläche 6 an die Auswerteeinheit 70 übermittelt. Falls die Kamera 42 nur dann ein Bild erzeugt, wenn sie einen Befehl von der Auswerteeinheit 70 erhalten hat, braucht die Kamera 42 nur so viele Bilder wie benötigt zu erzeugen, und nur so viele Bilder wie benötigt brauchen an die Auswerteeinheit 70 übermittelt zu werden. Dies spart Energie und Speicherplatz und manchmal auch Bandbreite ein.

Die Auswerte-Software 71 wertet automatisch übermittelte Bilder aus, welche die Kamera 42 erzeugt hat. Die Auswerte-Software 71 ermittelt anhand des QR-Codes 8 die eindeutige Kennung für die Sensoreinheit 10. Durch Auswertung der übermittelten Bilder entscheidet die Auswerte-Software 71 automatisch, ob eine ausreichende Menge einer Speichelprobe in den Probensammler 1 eingegeben wurde, ob die Probeneinheit 10 ein gültiges und verwertbares Auswertungsergebnis erzielt hat und ob die Speichelprobe mindestens eine der sechs oben genannten Substanzen in einer Konzentration oberhalb der Nachweisgrenze enthält oder nicht. In einer Ausgestaltung prüft die Auswerte-Software 71 durch Bildauswertung, wie schnell Streifen auf der Anzeigefläche 6 entstehen oder verschwinden. Bevorzugt wird eine Abfolge von Bildern ausgewertet. Die Geschwindigkeit der Veränderung korreliert mit einer Konzentration einer nachzuweisenden Dosis.

Bevorzugt wird auf dem Bildschirm 77 angezeigt, welche Ergebnisse die Auswerte-Software 71 erzielt hat.

In einer Ausgestaltung speichert die Auswerteeinheit 70 die Untersuchungsergebnisse für einen Probanden im Datenspeicher 76 ab, bevorzugt zusammen mit der Kennung (QR-Code) 8 für die Sensoreinheit 10 und einem Zeitstempel, wann die Sensoreinheit 10 die Speichelprobe des Probanden untersucht hat. Bevorzugt speichert die Auswerteeinheit 70 im Datenspeicher 76 zusätzlich den empfangenen zeitlichen Verlauf der Beschleunigung und die Dauer der Ruhephase ab, damit bei Bedarf später nachgewiesen werden kann, dass die Untersuchungsergebnisse der Sensoreinheit 10 gültig sind. In einer Ausgestaltung übermittelt die Kommunikationseinheit 72 die Untersuchungsergebnisse an einen räumlich entfernten Empfänger.

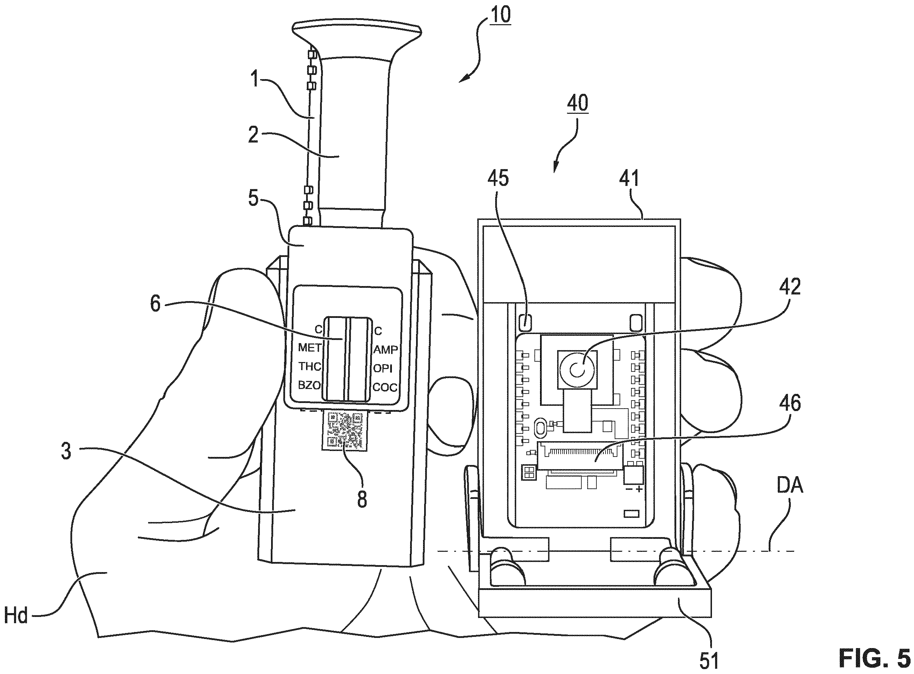

Figur 5 zeigt die Mess-Anordnung 10, 40 in einer Hand Hd eines Benutzers. Die Anzeigefläche 6 der Probeneinheit 10 sowie die Kamera 42 der Überwachungseinheit 40 zeigen zum Betrachter hin. Die Abdeckung 4 vor der Anzeigefläche 6 wurde entfernt. Der Probensammler 1 befindet sich hinter der Aufnahmeeinheit 2.

In der Ausgestaltung, die in Figur 3, Figur 4 und in Figur 5 gezeigt wird, umfasst die Überwachungseinheit 40 einen Ein- und Ausschalter sowie eine Meldeleuchte für die Anzeige, ob Überwachungseinheit 40 eingeschaltet oder ausgeschaltet ist. Möglich ist auch, dass die Überwachungseinheit 40 dadurch eingeschaltet wird, dass die Halteplatte 51 um die Drehachse DA vom Grundkörper 41 weggeklappt wird, und dadurch wieder ausgeschaltet wird, dass die Halteplatte 51 zum Grundkörper 41 hin gedreht wird (in die Transportposition). Darüber hinaus hat die Überwachungseinheit 40 keine Anzeigefläche und kann somit weder Meldungen an einen Menschen ausgeben noch Benutzereingaben empfangen. In vielen Fällen spart diese Ausgestaltung Platz, Gewicht sowie elektrische Energie ein. Die Überwachungseinheit 40 lässt sich nach einer Anwendung relativ leicht desinfizieren.

In einer nicht gezeigten Abwandlung umfasst die Überwachungseinheit 40 zusätzlich eine Anzeigeeinheit, die anzeigt, ob die verbundene Sensoreinheit 10 ein gültiges Untersuchungsergebnis erzielt hat und ob dieses Untersuchungsergebnis positiv (mindestens eine vorgegebene Substanz vorhanden) oder negativ ist. Optional umfasst die Überwachungseinheit 40 eine Meldeleuchte, die anzeigt, ob eine Kommunikationsverbindung mit der Auswerteeinheit 70 aktuell möglich ist oder nicht.

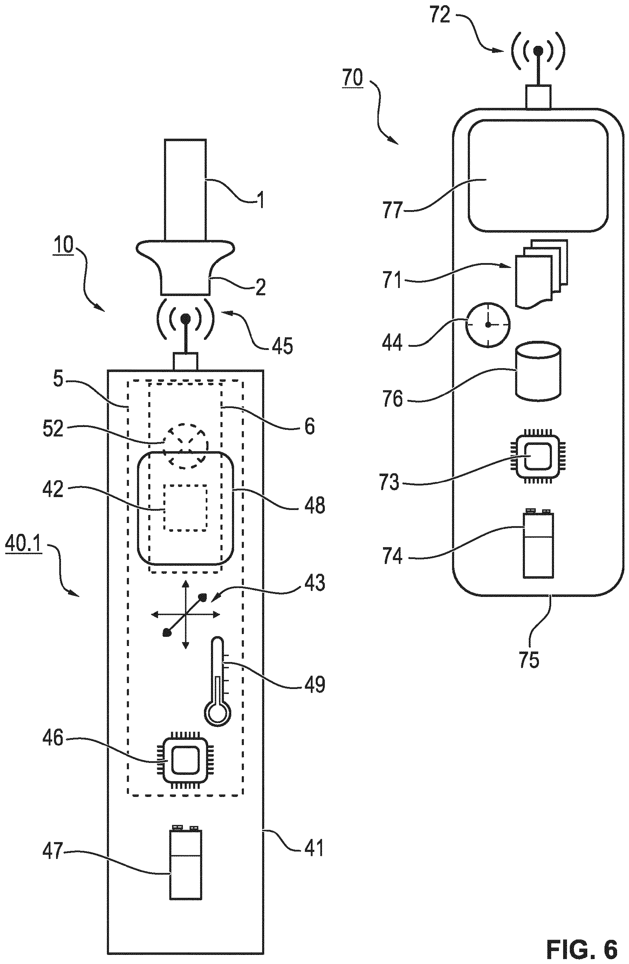

Figur 6 zeigt eine abweichende Ausgestaltung einer erfindungsgemäßen Überwachungseinheit, die als Überwachungseinheit 40.1 bezeichnet wird und die einen eigenen Bildschirm 48 aufweist. Gleiche Bezugszeichen haben die gleiche Bedeutung wie in Figur 3 bis Figur 5.

Die Überwachungseinheit 40.1 ist wegen des Bildschirms 48 etwas höher als die Überwachungseinheit 40 von Figur 3 bis Figur 5. Der Betrachter von Figur 6 schaut auf den Bildschirm 48 der Überwachungseinheit 40.1 sowie auf die Anzeigefläche 6 der Sensoreinheit 10. In der Betrachtungsrichtung von Figur 6 befindet die Sensoreinheit 10 sich hinter der Überwachungseinheit 40.1. Die Auswerte-Software 71 kann auf der Überwachungseinheit 40.1 installiert sein oder auf der separaten Auswerteeinheit 70 von Figur 3 und Figur 6. Bei der Ausgestaltung gemäß Figur 6 kann der Prüfer auf dem Bildschirm 48 der Überwachungseinheit 40.1 ablesen, ob er die Mess-Anordnung 10, 40.1 korrekt geschüttelt und anschließend richtig ruhig gehalten hat oder nicht. Bevorzugt wird mithilfe der Kommunikationseinheiten 45 und 72 eine drahtlose Kommunikationsverbindung zwischen der Überwachungseinheit 40.1 und der räumlich entfernten Auswerteeinheit 70 hergestellt.

Bei den Ausgestaltungen gemäß Figur 3 bis Figur 6 werden eine Überwachungseinheit 40, 40.1 mit einer Kamera 42 und einem Beschleunigungs-Sensor 43 sowie eine räumlich entfernte Auswerteeinheit 70 mit der Auswerte-Software 71 verwendet. Der Prüfer hält die Mess-Anordnung 40, 10 bzw. 40.1, 10 in der Hand und schüttelt sie. Möglich ist auch, dass die Überwachungseinheit mit der Kamera und dem Beschleunigungs-Sensor 43 nach Art einer Armbanduhr oder einer Smart Watch am Handgelenk getragen werden kann. Derartige Überwachungseinheiten sind beispielsweise für Sportler bekannt.

Der Prüfer nimmt die Sensoreinheit 10 mit der Speichelprobe in eine Hand und schüttelt sie. An dieser Hand trägt der Prüfer die Überwachungseinheit in Form einer Armbanduhr. Nach dem Schütteln und der Ruhepause bringt der Prüfer die Hand mit der Überwachungseinheit so in die Nähe der Sensoreinheit 10, dass die Kamera der Überwachungseinheit auf die Anzeigefläche 6 der Sensoreinheit 10 gerichtet ist. Auch in diesem Falle ist die Überwachungseinheit mit der räumlich entfernten Auswerteeinheit 70 über eine drahtlose Kommunikationsverbindung verbunden, z.B. per Bluetooth Low Energy.

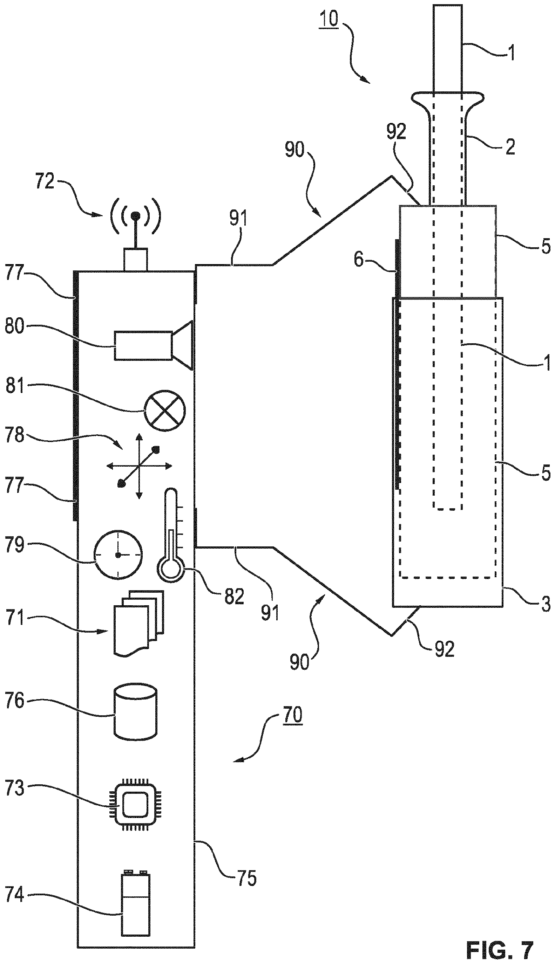

Figur 7 zeigt eine abweichende Ausgestaltung, bei der die Auswerteeinheit 70 zusätzlich als Überwachungseinheit verwendet wird. Im gezeigten Beispiel hat die Auswerteeinheit 70 die Form eines Smartphones. Gleiche Bezugszeichen haben die gleiche Bedeutung wie in Figur 3 bis Figur 5. Die Auswerteeinheit 70 von Figur 7 hat also genauso wie die Auswerteeinheit 70 von Figur 3 einen Bildschirm 77 und eine Kommunikationseinheit 72 am Gehäuse 75 sowie einen Datenspeicher 76, ein Steuergerät 73 und eine Spannungsversorgungseinheit 74 im Gehäuse 75. Wiederum ist die Auswerte-Software 71 auf einem Prozessor mit Datenspeicher der Auswerteeinheit 70 implementiert und wird von diesem Prozessor ausgeführt oder ist auf dem Prozessor ausführbar. In Figur 7 wird die Auswerteeinheit 70 von der Seite gezeigt, wobei der Bildschirm 77 nach links zeigt und die Fläche des Bildschirms 77 senkrecht auf der Zeichenebene von Figur 7 steht. Außerdem umfasst die Auswerteeinheit 70 von Figur 7 weitere Bestandteile, die ein übliches Smartphone häufig aufweist, nämlich eine Kamera 80, eine Lichtquelle 81, einen Beschleunigungs-Sensor 78 und eine Uhr 79. Optional umfasst die Überwachungseinheit 70 von Figur 7 ein Thermometer 82.

Die Sensoreinheit 10 lässt sich lösbar mit der Auswerteeinheit 70 verbinden, und zwar mithilfe eines röhrenförmigen Adapters 90. Der Adapter 90 umfasst eine sensorseitige Koppelstelle 92 und eine überwachungsseitige Koppelstelle 91. Die Sensoreinheit 10 lässt sich lösbar mit der sensorseitigen Koppelstelle 92 verbinden, die Auswerteeinheit 70 lösbar mit der überwachungsseitigen Koppelstelle 91.

Falls die Sensoreinheit 10 mit der Speichelprobe mithilfe des Adapters 90 mit der Auswerteeinheit 70 verbunden ist, wird wiederum eine Mess-Anordnung 10, 70, 90 gebildet, welche der Prüfer schütteln kann. Der Adapter 90 hält die Sensoreinheit 10 an der Auswerteeinheit 70, sodass der Prüfer die Auswerteeinheit 70 schütteln kann, ohne dass die Sensoreinheit 10 abfällt oder sich relativ zur Auswerteeinheit 70 bewegt. Die Kamera 80 der Auswerteeinheit 70 ist auf die Anzeigefläche 6 der Sensoreinheit 10 gerichtet. Der Adapter 90 umschließt lichtdicht einen Raum zwischen der Kamera 80 und der Anzeigefläche 6 und verhindert, dass in größerem Umfange Licht von außen in diesen Raum einfällt. Die optionale Lichtquelle 81 der Auswerteeinheit 70 beleuchtet diesen Raum von innen. Dadurch vermag die Auswerte-Software 71 auch bei wechselnden Lichtverhältnissen die Bilder von der Kamera 80 auszuwerten und dadurch die Untersuchungsergebnisse der Sensoreinheit 10 zu ermitteln.

Bevorzugt lässt sich der Adapter 90 wahlweise in einen Einstellungsmodus und in einen Betriebsmodus verbringen. Im Einstellungsmodus lässt sich die sensorseitigen Koppelstelle 92 relativ zu der überwachungsseitigen Koppelstelle 91 verschieben. Dadurch lässt sich der Adapter 90 so ausgestalten, dass die Kamera 80 der Überwachungseinheit 70 auf die Anzeigefläche 6 der Sensoreinheit 10 gerichtet ist. Diese Ausgestaltung ermöglicht es, denselben Adapter 90 für unterschiedliche Überwachungseinheiten 70 zu verwenden. In Betriebsmodus sind die beiden Koppelstelle 91 und 92 relativ zueinander fixiert, sodass die Sensoreinheit 10 mithilfe des Adapters 90 in einer bestimmten Position und Orientierung relativ zu der Überwachungseinheit 70 gehalten wird.

Ein derartiger Adapter 90, der einen Raum lichtdicht umschließt, lässt sich auch für die Ausgestaltung gemäß Figur 3 und Figur 4 verwenden.

Die Ausgestaltung, dass die Auswerteeinheit 70 zugleich als Überwachungseinheit fungiert, erspart die Notwendigkeit, eine Datenverbindung zwischen der Überwachungseinheit und der Auswerteeinheit herzustellen.

### Bezugszeichenliste

| | |
|---|---|
| 1 | hohler Probensammler der Sensoreinheit 10, nimmt eine Probe eines Probanden auf, lässt sich in die Aufnahmeeinheit 2 einsetzen |
| 2 | röhrenförmige Aufnahmeeinheit für den Probensammler 1, mit der Kassette 5 verbunden |
| 3 | Gehäuse der Sensoreinheit 10, umfasst die Abdeckung 4 |
| 4 | Abdeckung für die Anzeigefläche 6, gehört zum Gehäuse 3 |
| 5 | Kassette der Sensoreinheit 10, nimmt ein Gemisch aus der Probe und dem Reagens sowie sechs Indikator-Papierstreifen auf |
| 6 | Anzeigefläche der Kassette 5, hinter der Untersuchungsergebnisse (Verfärbungen) der Indikator-Papierstreifen 5 sichtbar sind |
| 8 | individuelle Kennung der Sensoreinheit 10, hat die Form eines QR-Codes |
| 9 | Aufnahmetasche am Gehäuse 3, nimmt vor einem Einsatz den Probensammler 1 auf |
| 10 | Sensoreinheit, umfasst die Kassette 5 mit der Anzeigefläche 6, das Gehäuse 3, die Aufnahmeeinheit 2, den Probensammler 1 und die Aufnahmetasche 9 |
| 20 | Sammeleinheit, umfasst den Probensammler 21, die Aufnahmeeinheit 22 und das Gehäuse 23 |
| 21 | Probensammler der Sammeleinheit 20 |
| 22 | Aufnahmeeinheit der Sammeleinheit 20, nimmt den Probensammler 21 auf |
| 23 | Gehäuse der Sammeleinheit 20, enthält eine Kammer für eine Speichelprobe |
| 24 | Kappe auf dem Probensammler 21 |
| 30 | stationäre Basisstation, vermag die Sammeleinheit 20 aufzunehmen, umfasst die Öffnung 31, die Anzeigeeinheit 36, die Bedienelemente 32 und im Inneren einen nicht gezeigten Sensor |
| 31 | verschließbare Öffnung der Basisstation 30, durch welche hindurch sich die Sammeleinheit 20 mit einer Speichelprobe hineinschieben lässt |
| 32 | Bedienelemente der Basisstation 30 |
| 36 | Anzeigeeinheit der Basisstation 30 |
| 40 | mobile Überwachungseinheit, umfasst das Gehäuse 41, die Kamera 42, das Betätigungselement 53, den Beschleunigungs-Sensor 43, die Kommunikationseinheit 45, das Thermometer 49, das Steuergerät 46 und die Spannungsversorgungseinheit 47 |
| 40.1 | mobile Überwachungseinheit, umfasst die gleichen Bestandteile wie die Überwachungseinheit 40 und zusätzlich den Bildschirm 48 |
| 41 | Gehäuse der Überwachungseinheit 40, umschließt den Grundkörper |
| 42 | Kamera der Überwachungseinheit 40, 40.1, in das Gehäuse 41 eingesetzt, auf die Anzeigefläche 6 der Sensoreinheit 10 gerichtet |
| 43 | Beschleunigungs-Sensor der Überwachungseinheit 40, 40.1 |
| 44 | Uhr der Auswerteeinheit |
| 45 | Kommunikationseinheit der Überwachungseinheit 40, 40.1 |
| 46 | Steuergerät der Überwachungseinheit 40, 40.1 |
| 47 | Spannungsversorgungseinheit der Überwachungseinheit 40, 40.1 |
| 48 | Bildschirm der Überwachungseinheit 40.1 |
| 49 | optionales Thermometer der Überwachungseinheit 40, 40.1 |
| 51 | verschwenkbare rechteckige Halteplatte der Überwachungseinheit 40, trägt die Sensoreinheit 10 |
| 52 | optionale Lichtquelle der Überwachungseinheit 40, 40.1, beleuchtet die Anzeigefläche 6 |
| 53 | optionales Betätigungselement zum manuellen Fokussieren der Kamera 42 |
| 70 | Auswerteeinheit, auf einem Smartphone realisiert, umfasst die Auswerte-Software 71, die Kommunikationseinheit 72, den Datenspeicher 76, die Uhr 44, das Steuergerät 73 und die Spannungsversorgungseinheit 74 |
| 71 | auf einem Rechner ausführbare Auswerte-Software der Auswerteeinheit 70, empfängt ein Bewegungs-Signal von dem Beschleunigungs-Sensor 43, 78 und entscheidet automatisch, ob die Sensoreinheit 10 korrekt bewegt und gehalten wurde |
| 72 | Kommunikationseinheit der Auswerteeinheit 70 |
| 73 | Steuergerät der Auswerteeinheit 70 |
| 74 | Spannungsversorgungseinheit der Auswerteeinheit 70 |
| 75 | Gehäuse der Auswerteeinheit 70 |
| 76 | Datenspeicher der Auswerteeinheit 70 |
| 77 | Bildschirm der Auswerteeinheit 70, in das Gehäuse 75 eingelassen |
| 78 | Beschleunigungs-Sensor der Auswerteeinheit 70, erzeugt ein Bewegungs-Signal |
| 79 | Uhr der Auswerteeinheit 70 |
| 80 | Kamera der Auswerteeinheit 70, in das Gehäuse 75 eingelassen |
| 81 | Lichtquelle der Auswerteeinheit 70, beleuchtet die Anzeigefläche 6 |
| 82 | optionales Thermometer der Auswerteeinheit 70 |
| 90 | Adapter, mit dem sich die Sensoreinheit 10 dergestalt an der Überwachungseinheit 70 verbinden lässt, dass die Kamera 60 auf die Anzeigefläche 6 gerichtet ist, umfasst die Koppelstellen 91 und 92 |
| 91 | überwachungsseitige Koppelstelle des Adapters 90, lässt sich lösbar mit der Überwachungseinheit 70 verbinden |
| 92 | sensorseitige Koppelstelle des Adapters 90, lässt sich lösbar mit der Sensoreinheit 10 verbinden |
| DA | Drehachse, um welche die Halteplatte 51 relativ zum Grundkörper mit dem Gehäuse 41 drehbar ist |
| Hd | Hand eines Benutzers, die die Mess-Anordnung 10, 40 hält |

## Patentansprüche

1. Anordnung zum Untersuchen einer Probe auf mindestens eine Substanz, wobei die Anordnung
- eine Sensoreinheit (10),
- eine Überwachungseinheit (40, 40.1, 70) mit einem ersten Überwachungs-Sensor (43, 78) und
- ein auf einem Rechner ausführbares Auswerte-Programm (71) umfasst,
wobei die Sensoreinheit (10)
- eine Eingabeeinheit (1) und
- einen Substanz-Sensor (5)
umfasst,
wobei die Eingabeeinheit (1)
- die Eingabe einer zu untersuchenden Probe ermöglicht und
- eine eingegebene Probe aufzunehmen vermag,
wobei der Substanz-Sensor (5)
- mindestens ein chemisches Reagens und
- mindestens einen Indikator (6)
umfasst,
wobei das oder jedes Reagens
- jeweils mindestens einer Substanz zugeordnet ist und
- mit einer von der Eingabeeinheit (1) aufgenommenen Probe chemisch zu reagieren vermag,
wobei die chemische Reaktion des oder jedes Reagens von dem Vorhandensein und / oder der Konzentration der oder jeder jeweils zugeordneten Substanz in einer von der Eingabeeinheit (1) aufgenommenen Probe abhängt,
wobei der oder jeder Indikator (6) dazu ausgestaltet ist, das jeweilige Ergebnis jeweils einer chemischen Reaktion visuell anzuzeigen,
wobei die Sensoreinheit (10) sich dergestalt mit der Überwachungseinheit (40, 40.1, 70) verbinden lässt, dass nach Herstellung der Verbindung die Sensoreinheit (10) keine Bewegung relativ zur Überwachungseinheit (40, 40.1, 70) auszuführen vermag, und
wobei der erste Überwachungs-Sensor (43, 78) dazu ausgestaltet ist, ein Maß für eine Bewegung der mit der Überwachungseinheit (40, 40.1, 70) verbundenen Sensoreinheit (10) im Raum automatisch zu messen, und
wobei das Auswerte-Programm (71) dazu ausgestaltet ist, durch Auswertung von Messwerten des ersten Überwachungs-Sensors (43, 78) automatisch zu entscheiden, ob der tatsächliche zeitliche Verlauf der Bewegung der Sensoreinheit (10) im Raum bis auf eine vorgegebene Toleranz gleich einem vorgegebenen zeitlichen Soll-Verlauf ist.

2. Anordnung nach Anspruch 1,
**dadurch gekennzeichnet, dass**
nach Herstellung der Verbindung zwischen der Sensoreinheit (10) und der Überwachungseinheit (40, 40.1, 70) eine tragbare Mess-Anordnung gebildet ist, die ein Mensch schütteln kann,
bevorzugt eine Mess-Anordnung, die ein Mensch in einer Hand (Hd) halten und schütteln kann.

3. Anordnung nach einem der vorhergehenden Ansprüche,
**dadurch gekennzeichnet, dass**
die Anordnung einen mechanischen Adapter (90) umfasst, ,
wobei die Sensoreinheit (10) mithilfe des Adapters (90) lösbar mit der Überwachungseinheit (40, 40.1, 70) verbunden oder verbindbar ist und
wobei bevorzugt als die Überwachungseinheit (40, 40.1, 70) ein tragbarer Rechner (70), insbesondere ein Smartphone, verwendet wird.

4. Anordnung nach Anspruch 3,
**dadurch gekennzeichnet, dass**
der Adapter (90)
- eine überwachungsseitige Koppelstelle (91), die sich lösbar mit dem tragbaren Rechner (70) verbinden lässt, und
- eine sensorseitige Koppelstelle (92), die sich lösbar mit der Sensoreinheit (10) verbinden lässt,
umfasst,
wobei der Adapter (90) so ausgestaltet ist, dass
- in einem Einstellungsmodus die eine Koppelstelle (92) relativ zu der anderen Koppelstelle (91) beweglich ist und
- in einem Betriebsmodus die eine Koppelstelle (92) relativ zu der anderen Koppelstelle (91) fixiert ist.

5. Anordnung nach einem der vorhergehenden Ansprüche,
**dadurch gekennzeichnet, dass**
der erste Überwachungs-Sensor einen Beschleunigungs-Sensor (43, 78) umfasst,
wobei der Beschleunigungs-Sensor (43, 78) den zeitlichen Verlauf einer auf die Überwachungseinheit (40, 40.1, 70) einwirkenden Beschleunigung im Raum zu messen vermag.

6. Anordnung nach einem der vorhergehenden Ansprüche,
**dadurch gekennzeichnet, dass**
das Auswerte-Programm (71) dazu ausgestaltet ist, eine Ausgabeeinheit (48, 77) dergestalt anzusteuern, dass die angesteuerte Ausgabeeinheit (48, 77) eine Information in einer von einem Menschen wahrnehmbaren Form auszugeben vermag,
wobei das Auswerte-Programm (71) dazu ausgestaltet ist,
- abhängig von Messwerten des ersten Überwachungs-Sensors (43, 78) eine Information über eine geforderte weitere Bewegung oder Beendigung einer weiteren Bewegung der Sensoreinheit (10) zu erzeugen und
- die Ausgabeeinheit (48, 77) derart anzusteuern, dass die angesteuerte Ausgabeeinheit (48, 77) die erzeugte Information ausgibt.

7. Anordnung nach einem der vorhergehenden Ansprüche,
**dadurch gekennzeichnet, dass**
der oder ein Überwachungs-Sensor ein Thermometer (49) umfasst,
wobei das Thermometer (49) dazu ausgestaltet ist, ein Maß für die Temperatur in der Umgebung der Überwachungseinheit (40, 40.1, 70) zu messen.

8. Anordnung nach Anspruch 7,
**dadurch gekennzeichnet, dass**
die Überwachungseinheit (40.1, 70) eine Ausgabeeinheit (48), insbesondere einen Bildschirm, umfasst,
wobei die Überwachungseinheit (40.1, 70) dazu ausgestaltet ist,
- abhängig von der gemessenen Umgebungstemperatur eine Nachricht zu erzeugen und
- die erzeugte Nachricht auf der Ausgabeeinheit (48) in einer von einem Menschen wahrnehmbaren Form auszugeben, und
wobei die erzeugte Nachricht eine geforderte Behandlung der Sensoreinheit (10) oder eine Gültigkeit eines Ergebnisses der Sensoreinheit (10) spezifiziert.

9. Anordnung nach einem der vorhergehenden Ansprüche,
**dadurch gekennzeichnet, dass**
die Überwachungseinheit (40, 40.1)
- einen Grundkörper (41) und
- eine mit dem Grundkörper (41) mechanisch verbundene Halteplatte (51) umfasst,
wobei die Sensoreinheit (10) sich auf die Halteplatte (51) stellen lässt und sich dann neben dem Grundkörper (41) befindet und
wobei bevorzugt die Halteplatte (51) schwenkbar mit dem Grundkörper (41) verbunden ist.

10. Verwendung einer Anordnung nach einem der vorhergehenden Ansprüche
zur Untersuchung einer Speichelprobe auf mindestens eine Droge oder ein Medikament oder auf mindestens einen Krankheitserreger oder einen Antikörper gegen einen Krankheitserreger.

11. Verfahren zum Untersuchen einer Probe auf mindestens eine Substanz,
wobei das Verfahren unter Verwendung einer Anordnung durchgeführt wird, welche
- eine Sensoreinheit (10),
- eine Überwachungseinheit (40, 40.1, 70) mit einem ersten Überwachungs-Sensor (43, 78) und
- ein auf einem Rechner ausführbares Auswerte-Programm (71) umfasst,
wobei die Sensoreinheit (10)
- eine Eingabeeinheit (1) und
- einen Substanz-Sensor (5)
umfasst,
wobei der Substanz-Sensor (5)
- mindestens ein chemisches Reagens und
- mindestens einen Indikator (6)
umfasst,
wobei das oder jedes Reagens jeweils mindestens einer Substanz zugeordnet ist und
wobei das Verfahren die Schritte umfasst, dass
- die Eingabeeinheit (1) eine in die Eingabeeinheit (1) eingegebene Probe aufnimmt,
- die Sensoreinheit (10) mit der aufgenommenen Probe dergestalt mit der Überwachungseinheit (40, 40.1, 70) verbunden wird, dass nach Herstellung der Verbindung die Sensoreinheit (10) keine Bewegung relativ zur Überwachungseinheit (40, 40.1, 70) auszuführen vermag,
- die Sensoreinheit (10) bewegt, insbesondere geschüttelt wird,
- das oder mindestens ein, bevorzugt jedes Reagens mit der von der Eingabeeinheit (1) aufgenommen Probe chemisch reagiert,
wobei die chemische Reaktion des oder jedes Reagens von dem Vorhandensein und / oder der Konzentration der oder jeder jeweils zugeordneten Substanz in der von der Eingabeeinheit (1) aufgenommenen Probe abhängt,
- der oder jeder Indikator (6) das jeweilige Ergebnis jeweils einer chemischen Reaktion visuell anzeigt,
- der erste Überwachungs-Sensor (43, 78) automatisch ein Maß für eine Bewegung der mit der Überwachungseinheit (40, 40.1, 70) verbundenen Sensoreinheit (10) im Raum misst und
- das Auswerte-Programm (71) durch Auswertung von Messwerten des ersten Überwachungs-Sensors (43, 78) automatisch entscheidet, ob der tatsächliche zeitliche Verlauf der Bewegung der Sensoreinheit (10) im Raum bis auf eine vorgegebene Toleranz gleich einem vorgegebenen zeitlichen Soll-Verlauf ist.

12. Verfahren nach Anspruch 11,
**dadurch gekennzeichnet, dass**
der Schritt, die Sensoreinheit (10) mit der Überwachungseinheit (40, 40.1, 70) zu verbinden, durchgeführt wird, bevor die Sensoreinheit (10) bewegt wird,
wobei die Überwachungseinheit (40, 40.1, 70) und die mit der Überwachungseinheit (40, 40.1, 70) verbundene Sensoreinheit (10) gemeinsam bewegt werden und
wobei der Schritt, dass der erste Überwachungs-Sensor (43, 78) ein Maß für eine Bewegung der Sensoreinheit (10) erfasst, den Schritt umfasst, dass
der Bewegungs-Sensor (43, 78) den zeitlichen Verlauf einer Bewegung der Überwachungseinheit (40, 40.1, 70), insbesondere einer auf die Überwachungseinheit (40, 40.1, 70) einwirkenden Beschleunigung, im Raum misst, während die Sensoreinheit (10) mit der Überwachungseinheit (40, 40.1, 70) verbunden ist.

## Claims

1. Arrangement for testing a sample for at least one substance, wherein the arrangement comprises
- a sensor unit (10),
- a monitoring unit (40, 40.1, 70) having a first monitoring sensor (43, 78), and
- an evaluation program that can be run on a computer (71),
wherein the sensor unit (10) comprises
- an input unit (1) and
- a substance sensor (5),
wherein the input unit (1)
- allows the input of a sample to be tested and
- is capable of accepting a submitted sample,
wherein the substance sensor (5) comprises
- at least one chemical reagent and
- at least one indicator (6),
wherein the or each reagent
- is assigned to at least one substance and
- is capable of chemically reacting with a sample taken up by the input unit (1),
wherein the chemical reaction of the or each reagent depends upon the presence and/or concentration of the or each assigned substance in a sample taken up by the input unit (1),
wherein the or each indicator (6) is designed to visually display the corresponding result of a chemical reaction,
wherein the sensor unit (10) can be connected to the monitoring unit (40, 40.1, 70) in such a way that, after the connection has been established, the sensor unit (10) is unable to perform any movement relative to the monitoring unit (40, 40.1, 70), and
wherein the first monitoring sensor (43, 78) is designed to automatically measure a degree of a movement of the sensor unit (10) connected to the monitoring unit (40, 40.1, 70) in space, and
wherein the evaluation program (71) is designed to automatically decide, by evaluating measured values of the first monitoring sensor (43, 78), whether the actual temporal course of the movement of the sensor unit (10) in space is equal to a predetermined temporal target course up to a predetermined tolerance.

2. Arrangement according to claim 1,
**characterized in that**,
after establishing the connection between the sensor unit (10) and the monitoring unit (40, 40.1, 70), a portable measuring arrangement is formed which a person can shake,
preferably a measuring arrangement that a person can hold and shake in one hand (Hd).

3. Arrangement according to any of the preceding claims,
**characterized in that**
the arrangement comprises a mechanical adapter (90),
wherein the sensor unit (10) is detachably connected or connectable to the monitoring unit (40, 40.1, 70) by means of the adapter (90) and
wherein a portable computer (70), in particular a smartphone, is preferably used as the monitoring unit (40, 40.1, 70).

4. Arrangement according to claim 3,
**characterized in that**
the adapter (90) comprises
- a monitoring-side coupling point (91) which can be detachably connected to the portable computer (70), and
- a sensor-side coupling point (92) which can be detachably connected to the sensor unit (10),
wherein the adapter (90) is designed such that,
- in one setting mode, one coupling point (92) is movable relative to the other coupling point (91), and,
- in one operating mode, one coupling point (92) is fixed relative to the other coupling point (91).

5. Arrangement according to any of the preceding claims,
**characterized in that**
the first monitoring sensor comprises an acceleration sensor (43, 78),
wherein the acceleration sensor (43, 78) is capable of measuring the temporal course of an acceleration in space acting upon the monitoring unit (40, 40.1, 70).

6. Arrangement according to any of the preceding claims,
**characterized in that**
the evaluation program (71) is designed to control an output unit (48, 77) in such a way that the controlled output unit (48, 77) is able to output information in a form perceptible by a human,
wherein the evaluation program (71) is designed,
- depending upon measured values of the first monitoring sensor (43, 78), to generate information about a required further movement or termination of a further movement of the sensor unit (10) and
- to control the output unit (48, 77) such that the controlled output unit (48, 77) outputs the generated information.

7. Arrangement according to any of the preceding claims,
**characterized in that**
the or a monitoring sensor comprises a thermometer (49),
wherein the thermometer (49) is designed to measure a degree of the temperature in the surroundings of the monitoring unit (40, 40.1, 70).

8. Arrangement according to claim 7,
**characterized in that**
the monitoring unit (40.1, 70) comprises an output unit (48), in particular a screen,
wherein the monitoring unit (40.1, 70) is designed
- to generate a message depending upon the measured temperature of the surroundings and
- to output the generated message on the output unit (48) in a form perceptible by a human, and
wherein the generated message specifies a required treatment of the sensor unit (10) or a validity of a result of the sensor unit (10).

9. Arrangement according to any of the preceding claims,
**characterized in that**
the monitoring unit (40, 40.1) comprises
- a base body (41) and
- a holding plate (51) mechanically connected to the base body (41),
wherein the sensor unit (10) can be placed on the holding plate (51) and is then located next to the base body (41) and
wherein the holding plate (51) is preferably pivotally connected to the base body (41).

10. Use of an arrangement according to any of the preceding claims
for testing a saliva sample for at least one drug or medication or for at least one pathogen or antibody against a pathogen.

11. Method for testing a sample for at least one substance,
wherein the method is carried out using an arrangement which comprises
- a sensor unit (10),
- a monitoring unit (40, 40.1, 70) having a first monitoring sensor (43, 78), and
- an evaluation program that can be run on a computer (71),
wherein the sensor unit (10) comprises
- an input unit (1) and
- a substance sensor (5),
wherein the substance sensor (5) comprises
- at least one chemical reagent and
- at least one indicator (6),
wherein the or each reagent is assigned to at least one substance and
wherein the method comprises the steps whereby
- the input unit (1) receives a sample submitted to the input unit (1),
- the sensor unit (10) having the taken-up sample is connected to the monitoring unit (40, 40.1, 70) in such a way that, after the connection has been established, the sensor unit (10) is unable to perform any movement relative to the monitoring unit (40, 40.1, 70),
- the sensor unit (10) is moved, in particular shaken,
- the or at least one, preferably each, reagent reacts chemically with the sample taken up by the input unit (1),
wherein the chemical reaction of the or each reagent depends upon the presence and/or concentration of the or each assigned substance in the sample taken up by the input unit (1),
- the or each indicator (6) visually displays the corresponding result of a chemical reaction,
- the first monitoring sensor (43, 78) automatically measures a degree of a movement of the sensor unit (10) connected to the monitoring unit (40, 40.1, 70) in space and
- the evaluation program (71) automatically decides, by evaluating measured values of the first monitoring sensor (43, 78), whether the actual temporal course of the movement of the sensor unit (10) in space is equal to a predetermined temporal target course up to a predetermined tolerance.

12. Method according to claim 11,
**characterized in that**
the step of connecting the sensor unit (10) to the monitoring unit (40, 40.1, 70) is carried out before the sensor unit (10) is moved,
wherein the monitoring unit (40, 40.1, 70) and the sensor unit (10) connected to the monitoring unit (40, 40.1, 70) are moved together and
wherein the step of the first monitoring sensor (43, 78) detecting a measure of a movement of the sensor unit (10) comprises the step whereby
the motion sensor (43, 78) measures the temporal course of a movement of the monitoring unit (40, 40.1, 70), in particular an acceleration acting upon the monitoring unit (40, 40.1, 70), in space, while the sensor unit (10) is connected to the monitoring unit (40, 40.1, 70).

## Revendications

1. Système pour l'analyse d'un échantillon concernant au moins une substance, dans lequel le système comprend
- une unité de détection (10),
- une unité de surveillance (40, 40.1, 70) comportant un premier détecteur de surveillance (43, 78) et
- un programme d'évaluation (71) exécutable sur un calculateur,
dans lequel l'unité de détection (10) comprend
- une unité d'admission (1) et
- un détecteur de substance (5),
dans lequel l'unité d'admission (1)
- permet l'admission d'un échantillon à analyser et
- est capable de recevoir un échantillon admis,
dans lequel le détecteur de substance (5) comprend
- au moins un réactif chimique et
- au moins un indicateur (6),
dans lequel le réactif ou chaque réactif
- est associé à respectivement au moins une substance et
- est capable de réagir chimiquement avec un échantillon reçu par l'unité d'admission (1),
dans lequel la réaction chimique du réactif ou de chaque réactif dépend de la présence et/ou de la concentration de la substance respectivement associée ou de chaque substance respectivement associée dans un échantillon reçu par l'unité d'admission (1),
dans lequel l'indicateur ou chaque indicateur (6) est configuré pour indiquer visuellement le résultat respectif de respectivement une réaction chimique,
dans lequel l'unité de détection (10) peut être reliée à l'unité de surveillance (40, 40.1, 70) de sorte qu'après l'établissement de la liaison, l'unité de détection (10) n'est pas capable d'exécuter de déplacement par rapport à l'unité de surveillance (40, 40.1, 70), et
dans lequel le premier détecteur de surveillance (43, 78) est configuré pour mesurer automatiquement une mesure d'un déplacement de l'unité de détection (10) reliée à l'unité de surveillance (40, 40.1, 70) dans l'espace, et
dans lequel le programme d'évaluation (71) est configuré pour décider automatiquement, par évaluation de valeurs de mesure du premier détecteur de surveillance (43, 78), si l'évolution dans le temps effective du déplacement de l'unité de détection (10) dans l'espace est égale, à une tolérance prédéfinie, à une évolution de consigne dans le temps prédéfinie.

2. Système selon la revendication 1,
**caractérisé en ce que**
après l'établissement de la liaison entre l'unité de détection (10) et l'unité de surveillance (40, 40.1, 70) est formé un système de mesure portable qu'une personne peut agiter,
de préférence, un système de mesure qu'une personne peut tenir dans une main (Hd) et agiter.

3. Système selon l'une des revendications précédentes,
**caractérisé en ce que**
le système comprend un adaptateur (90) mécanique,
dans lequel l'unité de détection (10) est reliée ou peut être reliée de manière amovible à l'unité de surveillance (40, 40.1, 70) à l'aide de l'adaptateur (90) et
dans lequel, de préférence, un calculateur portable (70), en particulier un mobile multifonction, est utilisé comme unité de surveillance (40, 40.1, 70).

4. Système selon la revendication 3,
**caractérisé en ce que**
l'adaptateur (90) comprend
- un point d'accouplement (91) côté surveillance qui peut être relié de manière amovible au calculateur portable (70), et
- un point d'accouplement (92) côté détecteur qui peut être relié de manière amovible à l'unité de détection (10),,
dans lequel l'adaptateur (90) est configuré de sorte que
- dans un mode de réglage, l'un des points d'accouplement (92) peut se déplacer par rapport à l'autre point d'accouplement (91), et
- dans un mode de fonctionnement, l'un des points d'accouplement (92) est fixe par rapport à l'autre point d'accouplement (91).

5. Système selon l'une des revendications précédentes,
**caractérisé en ce que**
le premier détecteur de surveillance comprend un détecteur d'accélération (43, 78),
dans lequel le détecteur d'accélération (43, 78) est capable de mesurer l'évolution dans le temps d'une accélération agissant sur l'unité de surveillance (40, 40.1, 70) dans l'espace.

6. Système selon l'une des revendications précédentes,
**caractérisé en ce que**
le programme d'évaluation (71) est configuré pour commander une unité d'émission (48, 77) de sorte que l'unité d'émission (48, 77) commandée est capable d'émettre une information sous une forme perceptible par une personne,
dans lequel le programme d'évaluation (71) est configuré pour
- générer, en fonction de valeurs de mesure du premier détecteur de surveillance (43, 78), une information sur un autre déplacement demandé ou sur un achèvement d'un autre déplacement de l'unité de détection (10) et
- commander l'unité d'émission (48, 77) de telle sorte que l'unité d'émission (48, 77) commandée émet l'information générée.

7. Système selon l'une des revendications précédentes,
**caractérisé en ce que**
le détecteur de surveillance ou un détecteur de surveillance comprend un thermomètre (49),
dans lequel le thermomètre (49) est conçu pour mesurer une mesure de la température dans l'environnement de l'unité de surveillance (40, 40.1, 70).

8. Système selon la revendication 7,
**caractérisé en ce que**
l'unité de surveillance (40.1, 70) comprend une unité d'émission (48), en particulier un écran,
dans lequel l'unité de surveillance (40.1, 70) est configurée pour
- générer un message en fonction de la température environnante mesurée et
- émettre le message généré sur l'unité d'émission (48) sous une forme perceptible par une personne, et
dans lequel le message généré spécifie un traitement demandé de l'unité de détection (10) ou une validité d'un résultat de l'unité de détection (10).

9. Système selon l'une des revendications précédentes,
**caractérisé en ce que**
l'unité de surveillance (40, 40.1) comprend
- un corps de base (41) et
- une plaque de maintien (51) reliée mécaniquement au corps de base (41),
dans lequel l'unité de détection (10) peut être placée sur la plaque de maintien (51) et se trouve alors à côté du corps de base (41) et
dans lequel, de préférence, la plaque de maintien (51) est reliée de manière pivotante au corps de base (41).

10. Utilisation d'un système selon l'une des revendications précédentes
pour l'analyse d'un échantillon de salive concernant au moins un stupéfiant ou un médicament ou concernant au moins un agent pathogène ou un anticorps dirigé contre un agent pathogène.

11. Procédé pour l'analyse d'un échantillon concernant au moins une substance,
dans lequel le procédé est mis en oeuvre en utilisant un système qui comprend
- une unité de détection (10),
- une unité de surveillance (40, 40.1, 70) comportant un premier détecteur de surveillance (43, 78) et
- un programme d'évaluation (71) exécutable sur un calculateur,
dans lequel l'unité de détection (10) comprend
- une unité d'admission (1) et
- un détecteur de substance (5),
dans lequel le détecteur de substance (5) comprend
- au moins un réactif chimique et
- au moins un indicateur (6),
dans lequel le réactif ou chaque réactif est associé à respectivement au moins une substance et
dans lequel le procédé comprend les étapes selon lesquelles
- l'unité d'admission (1) reçoit un échantillon admis dans l'unité d'admission (1),
- l'unité de détection (10) comportant l'échantillon reçu est reliée à l'unité de surveillance (40, 40.1, 70) de sorte qu'après l'établissement de la liaison, l'unité de détection (10) n'est pas capable d'exécuter de déplacement par rapport à l'unité de surveillance (40, 40.1, 70),
- l'unité de détection (10) est déplacée, en particulier agitée,
- le réactif ou au moins un réactif, de préférence chaque réactif réagit chimiquement avec l'échantillon reçu par l'unité d'admission (1),
dans lequel la réaction chimique du réactif ou de chaque réactif dépend de la présence et/ou de la concentration de la substance respectivement associée ou de chaque substance respectivement associée dans l'échantillon reçu par l'unité d'admission (1),
- le ou chaque indicateur (6) indique visuellement le résultat respectif de respectivement une réaction chimique,
- le premier détecteur de surveillance (43, 78) mesure automatiquement une mesure pour un déplacement de l'unité de détection (10) reliée à l'unité de surveillance (40, 40.1, 70) dans l'espace et
- le programme d'évaluation (71) décide automatiquement, par évaluation de valeurs de mesure du premier détecteur de surveillance (43, 78), si l'évolution dans le temps effective du déplacement de l'unité de détection (10) dans l'espace est égale, à une tolérance prédéfinie, à une évolution de consigne dans le temps prédéfinie.

12. Procédé selon la revendication 11,
**caractérisé en ce que**
l'étape dans laquelle l'unité de détection (10) est reliée à l'unité de surveillance (40, 40.1, 70) est mise en oeuvre avant que l'unité de détection (10) ne soit déplacée,
dans lequel l'unité de surveillance (40, 40.1, 70) et l'unité de détection (10) reliée à l'unité de surveillance (40, 40.1, 70) sont déplacées conjointement et
dans lequel l'étape dans laquelle le premier détecteur de surveillance (43, 78) détecte une mesure d'un déplacement de l'unité de détection (10) comprend l'étape dans laquelle
le détecteur de déplacement (43, 78) mesure l'évolution dans le temps d'un déplacement de l'unité de surveillance (40, 40.1, 70), en particulier d'une accélération agissant sur l'unité de surveillance (40, 40.1, 70), dans l'espace, tandis que l'unité de détection (10) est reliée à l'unité de surveillance (40, 40.1, 70).
